# EUROPEAN PATENT SPECIFICATION

(11) **EP 4 146 057 B1**
(45) Date of publication and mention of the grant of the patent: **09.04.2025**
(21) Application number: 21799487.0
(22) Date of filing: 27.04.2021
(51) Int. Cl.: A61K 9/00, A61K 9/70, A61M 5/00, A61K 31/704, A61K 33/24, A61B 5/00, A61M 5/32, A61M 37/00

(54) **BIOORTHOGONAL CATALYTIC PATCH AND METHODS OF USING THE SAME**
BIOORTHOGONALES KATALYTISCHES PFLASTER UND VERFAHREN ZUR VERWENDUNG DAVON
PATCH CATALYTIQUE BIOORTHOGONAL ET SES PROCÉDÉS D'UTILISATION

(30) Priority: 06.05.2020 US 202063021022 P
(43) Date of publication of application: 15.03.2023
(73) Proprietor: THE REGENTS OF THE UNIVERSITY OF CALIFORNIA, Oakland, CA 94607-5200 (US); North Carolina State University, Raleigh, North Carolina 27606 (US)
(72) Inventor: GU, Zhen, Los Angeles, California 90095-7191 (US); CHEN, Zhaowei, Los Angeles, California 90095-7191 (US)
(74) Representative: Ipsilon Benelux
(86) International application number: PCT/US2021/029381
(87) International publication number: WO 2021/225826

(56) References cited:
- WO-A1-2014/202994
- WO-A1-2019/075275
- WO-A1-2020/017441
- WO-A1-2020/179850
- CN-A- 107 349 518
- US-A1- 2018 177 990
- US-A1- 2019 201 675
- US-B1- 6 256 533
- US-B2- 10 064 950
- MILLER MILES A. ET AL: "Nano-palladium is a cellular catalyst for in vivo chemistry", NATURE COMMUNICATIONS, vol. 8, no. 1, 12 July 2017 (2017-07-12), pages 1 - 13, XP055980842, Retrieved from the Internet <URL:http://www.nature.com/articles/ncomms15906> DOI: 10.1038/ncomms15906
- P. LIU ET AL: "Photochemical route for synthesizing atomically dispersed palladium catalysts", SCIENCE, vol. 352, no. 6287, 12 May 2016 (2016-05-12), US, pages 797 - 800, XP055478351, ISSN: 0036-8075, DOI: 10.1126/science.aaf5251
- CAO YU ET AL: "TiO 2 Nanosheets with the Au Nanocrystal-Decorated Edge for Mitochondria-Targeting Enhanced Sonodynamic Therapy", CHEMISTRY OF MATERIALS, vol. 31, no. 21, 17 October 2019 (2019-10-17), US, pages 9105 - 9114, XP093085142, ISSN: 0897-4756, DOI: 10.1021/acs.chemmater.9b03430
- MILLER MILES A., MIKULA HANNES, LUTHRIA GAURAV, LI RAN, KRONISTER STEFAN, PRYTYSKACH MARK, KOHLER RAINER H., MITCHISON TIMOTHY, WE: "Modular Nanoparticulate Prodrug Design Enables Efficient Treatment of Solid Tumors Using Bioorthogonal Activation", ACS NANO, vol. 12, no. 12, 26 December 2018 (2018-12-26), US, pages 12814 - 12826, XP055870685, ISSN: 1936-0851, DOI: 10.1021/acsnano.8b07954
- JINQIANG WANG, YANQI YE, JICHENG YU, ANNA R. KAHKOSKA, XUDONG ZHANG, CHAO WANG, WUJIN SUN, RIA D. CORDER, ZHAOWEI CHEN,SAAD A. KHA: "Core-Shell Microneedle Gel for Self-Regulated Insulin Delivery", ACS NANO, vol. 12, no. 3, 27 March 2018 (2018-03-27), pages 2466 - 2473, XP055645230, ISSN: 1936-0851, DOI: 10.1021/acsnano.7b08152

## Description

### Technical Field

The technical field generally relates to biocompatible microneedles and patches containing such microneedles. More particularly, the technical field relates to a patch that incorporates microneedles that catalyze biorthogonal chemistry *in vivo* (and *in vitro*). More specifically, the technical field relates to patches that contain microneedles that are made of polyvinyl alcohol (PVA) matrix with TiO₂ nanosheet-supported palladium nanoparticles (Pd-TNSs) as the nanofillers.

### Background

Bioorthogonal catalysis has enabled a wealth of non-natural chemical transformations in complex living environs, such as cross-coupling and protective groupcleavage reactions for biomolecular labeling and protein/prodrug activation, broadening the understanding and interrogation of biological processes. The implementation of this class of chemistry mainly relies on abiotic transition metals (TMs) including Pd, Au, Ru, Ir and Cu, which are typically categorized into two formulations, metal complexes and metal nanoparticles. Because of their unpredicted toxicity, lack of target ability and limited stability, the application of metal complexes has been largely restricted to bacterial and cellular studies. With the aim to ameliorate the potential toxicity of TMs, researchers have shifted their attention to supported TM nanoparticles, mostly on resin beads, to modulate heterogenous bioorthogonal catalysis both inside and outside cells. By further transplanting resin-supported TMs into zebrafish embryos, orthogonal conversion of exogeneous substrates has been demonstrated, but the further application of such formulated TMs requires elaborate and complicated surgeries. Although progress has been made in improving the possibility of bioorthogonal catalysis *in vivo* by using injectable TMs, concerns still exist over how to withdraw TMs after treatment, potential metal toxicity, immunogenicity and unexpected catalysis caused by nonspecific deposition, not to mention the requirements for disparate dosage optimization of TMs and substrate molecules. In this line of reasoning, a bioorthogonal catalytic device that can work in a minimally invasive and spatially controlled manner would provide vast versatility for manipulating abiotic chemistry in higher-level living entities.

WO 2014/202994 A1 discloses a bioorthogonal deprotection method for preparing heterocyclic compounds by bond cleavage using palladium. The method has general application in the field of biological synthetic chemistry. Compounds, such as prodrugs, which are useful in such methods are also provided. While this provides technical advances, improvements remain desirable.

### Summary

Disclosed herein are microneedle-based devices (e.g., patches) that are a simple and robust device for catalyzing bioorthogonal chemistry both in *vitro* and *in vivo.* Here, a catalytically active microneedle-based patch device was manufactured made of polyvinyl alcohol (PVA) matrix with TiO₂ nanosheet-supported palladium nanoparticles (Pd-TNSs) as the nanofillers. The incorporation of the Pd-TNSs greatly enhanced the mechanical performance of the microneedles in the dry glass state, conferring on them sufficient strength to pierce into skin in a minimally invasive manner. Once placed in aqueous environment (e.g., within the skin tissue), the microneedles turned into a swollen hydrogel state, forming microporous structures. This construction has three levels of structural hierarchies, namely the three-dimensional needle array, the open micropores within in each needle matrix and the highly exposed Pd-TNSs surface in the network, which facilitate the diffusion of caged molecules to contact with the Pd nanoparticles and thereby promoted their activation. Remarkably, by using a mouse melanoma model, local intratumor activation of a systemically administered prodrug, N-(allyloxycarbonyl)doxorubicin, was demonstrated, which not only enabled increasing of dosing but also limited the side effects towards organs and tissues far away. Of note, the rich hydrogen bonds in the nanocomposites conferred the microneedle tips enough mechanical toughness in the hydrate state and made it easy to remove the whole patch, without leaving potentially hazardous transition metals within the body or inducing inflammation.

The invention provides a patch for *in vivo* biorthogonal catalysis applications in mammalian tissue includes a base or substrate having a plurality of microneedles extending away from the surface of the base, wherein the plurality of microneedles are formed from a polyvinyl alcohol (PVA) matrix with TiO₂ nanosheet-supported palladium nanoparticles (Pd-TNSs) dispersed therein. To use the patch, the patch is placed on living tissue of mammal such that the plurality of microneedles penetrate the tissue. This may be, for example, skin tissue although the patch may be applied to other tissue types. A prodrug that is administered to the mammal is then catalyzed by the Pd-TNSs in the patch device into a therapeutic agent or drug. In one embodiment, the prodrug comprises N-(allyloxycarbonyl)doxorubicin (allocDOX) and the produced therapeutic agent or drug is doxorubicin (DOX). Further developments are according to dependent claims 2-10.

A therapeutic system according to claim 11 is also provided that includes the microneedle patch and the prodrug which is delivered to the mammalian subject. Further developments are according to dependent claims 12-15.

While the prodrug used in the experiments was N-(allyloxycarbonyl)doxorubicin (alloc-DOX), it should be appreciated that other allyloxycarbonyl-modified prodrugs may also be used in a similar manner.

### Brief Description of the Drawings

FIG. 1A illustrates a plan view of a patch for *in vivo* biorthogonal catalysis applications according to one embodiment. The patch includes a base or substrate and a plurality of microneedles that extend from a surface thereof. The number of microneedles illustrated is illustrative as the number of actual microneedles in the patch may be tuned or adjusted as needed.
FIG. 1B illustrates a cross-sectional view of a patch illustrating the base or substrate and the plurality of microneedles that extend from a surface thereof.
FIG. 1C illustrates a patch for *in vivo* biorthogonal catalysis applications being applied to skin tissue of a mammal (e.g., human).
FIG. 1D schematically illustrates an embodiment of a nanofiller in the form of thin TiO₂ nanosheets (TNSs) that have one or more surfaces populated with metallic nanoparticles.
FIG. 2A illustrates a schematic of *in situ* bioorthogonal catalysis mediated by a microneedle patch for locally activating systematically administrated prodrugs *in vivo.* The microneedles are formed from a polyvinyl alcohol (PVA) matrix with TiO₂ nanosheet-supported palladium nanoparticles (Pd-TNSs) dispersed therein. The prodrug is activated by the Pd-TNSs into the therapeutic agent or drug.
FIG. 2B illustrates a transmission electron microscope (TEM) image of the TiO₂ nanosheets (TNSs). Scale bar: 200 nm.
FIG. 2C illustrates a TEM image Pd-deposited TNSs. Scale bar: 100 nm.
FIG. 2D illustrates scanning TEM/energy elemental mapping of Pd-TNS. Scale bar: 20 nm.
FIG. 2E illustrates Fourier transformed-EXAFS spectra of Pd-TNSs, Pd foil and PdO powder at the Pd *K*-edge.
FIG. 2F schematically illustrates decaging non-fluorescent alloc-RhB 110 into fluorescent RhB 110 (Eₓ=485 nm, Eₘ=520 nm).
FIG. 2G illustrates fluorescence intensity of the released RhB 110 in suspensions containing Pd-TNS plus alloc-RhB 110 and control groups with alloc-RhB 110 or Pd-TNS alone; inset: photographs of the corresponding reaction mixtures (after incubation for 24 h) under ultraviolet light. Data points represent mean ± s.d. *(n* = 3).
FIG. 3A illustrates a microneedle array patch (PT-MNs) that incorporates Pd-TNSs.
FIG. 3B illustrates a scanning electron microscope (SEM) image of a microneedle array patch (PT-MNs) that incorporates Pd-TNSs. Scale bar in: 500 µm.
FIG. 3C illustrates SEM images showing the longitudinal section of one microneedle (top, scale bar: 200 µm) and the distribution of Pd-TNSs inside the matrix (bottom, scale bar: 20 µm).
FIG. 3D illustrates a graph showing changes in fluorescence intensity of the decaged RhB 110 mediated by PT-MNs. Data points represent mean ± s.d. (*n* = 3). (i) The arrays of PT-MNs were immersed within the solution for 24 h; (ii) the arrays of PT-MNs were immersed within the solution for 6 h; (iii) pure PVA microneedles were utilized.
FIG. 3E illustrates SEM images of the lyophilized microneedles after swelling as viewed from the surface. The entrapment of Pd-TNSs within the pore walls were circled in red. Scale bar: 2 µm.
FIG. 3F illustrates SEM images of the lyophilized microneedles after swelling as viewed from the inner section. The entrapment of Pd-TNSs within the pore walls were circled in red. Scale bar: 2 µm.
FIG. 4A illustrates flow cytometry analysis of the B16F10 cells after different time treated with alloc-RhB 110 and PT-MNs (loaded with Pd-TNSs). This shows PT-MNs mediated bioorthogonal catalysis in cellular culture.
FIG. 4B illustrates flow cytometry analysis of the B16F10 cells after different time treated with alloc-RhB 110 alone.
FIG. 4C illustrates flow cytometry analysis of the B16F10 cells after different time treated with PT-MNs alone.
FIG. 4D illustrates a graph of the corresponding fluorescence intensities of the histograms of FIGS. 4A-4C.
FIG. 4E illustrates confocal laser microscopy images showing the fluorescence changes inside B16F10 cells after treated with alloc-RhB 110 and PT-MNs combination at different time points (scale bars, 50 µm).
FIG. 4F illustrates a graph showing fluorescence intensity as a function of distance at different time intervals along the indicated white dashed lines in the merged images of FIG. 4E. Corresponding to the flow cytometry analysis, time-dependent increase in fluorescence was directly observed for groups treated with alloc-RhB 110 and PT-MNs combination, while no obvious change was detected for control groups (FIGS. 4G-4H).
FIG. 4G illustrates confocal laser microscopy images showing the fluorescence changes inside B 16F 10 cells after treated with alloc-RhB 110 alone at different time points (scale bars, 50 µm).
FIG. 4H illustrates a graph showing fluorescence intensity as a function of distance at different time intervals along the indicated white dashed lines in the merged images of FIG. 4G.
FIG. 4I illustrates confocal laser microscopy images showing the fluorescence changes inside B16F10 cells after treated with PT-MNs alone at different time points (scale bars, 50 µm).
FIG. 4J illustrates a graph showing fluorescence intensity as a function of distance at different time intervals along the indicated white dashed lines in the merged images of FIG. 4I.

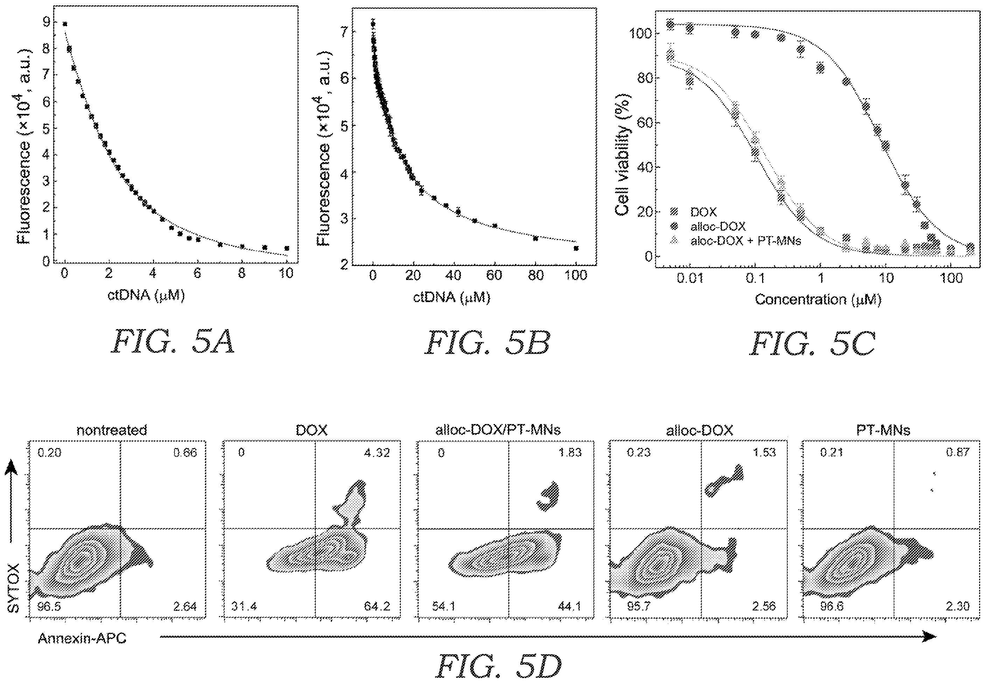

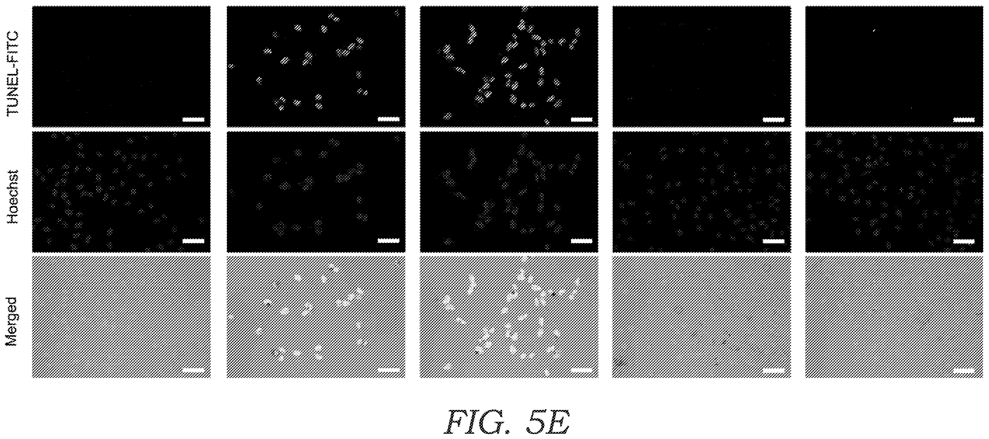

FIGS. 5A-5E illustrate the results of PT-MNs-mediated prodrug activation *in vitro.* FIGS. 5A, 5A illustrate spectrofluorometric titrations of ctDNA/DOX (FIG. 5A) and ctDNA/alloc-DOX (FIG. 5B) interactions. Data points represent mean ± s.d. (*n* = 3). FIG. 5C shows the viability of B 16F 10 cells treated with DOX, alloc-DOX and alloc-DOX/PT-MNs combination at different drug concentrations. Data points represent mean ± s.d. (*n =* 3). FIG. 5D illustrates flow cytometry analysis of the B16F10 cell apoptosis at 12 h after treated with normal culture, DOX, alloc-DOX/PT-MNs combination, alloc-DOX and PT-MNs, respectively, using the Annexin V-APC (which binds to the externalized phosphatidylserine upon the initiation of apoptosis) and SYTOX Green (which stains the nucleic acids of necrotic and apoptotic cells with compromised membranes). FIG. 5E illustrates the laser scanning confocal microscopy images of B16F10 cells stained with FITC-dUTP (green, TUNEL assay) and Hoechst 33342 (blue, nuclei staining), corresponding to the groups shown in panel (FIG. 5D). The appearance of green in the nuclei indicates cellular apoptosis. Scale bar: 50 µm.

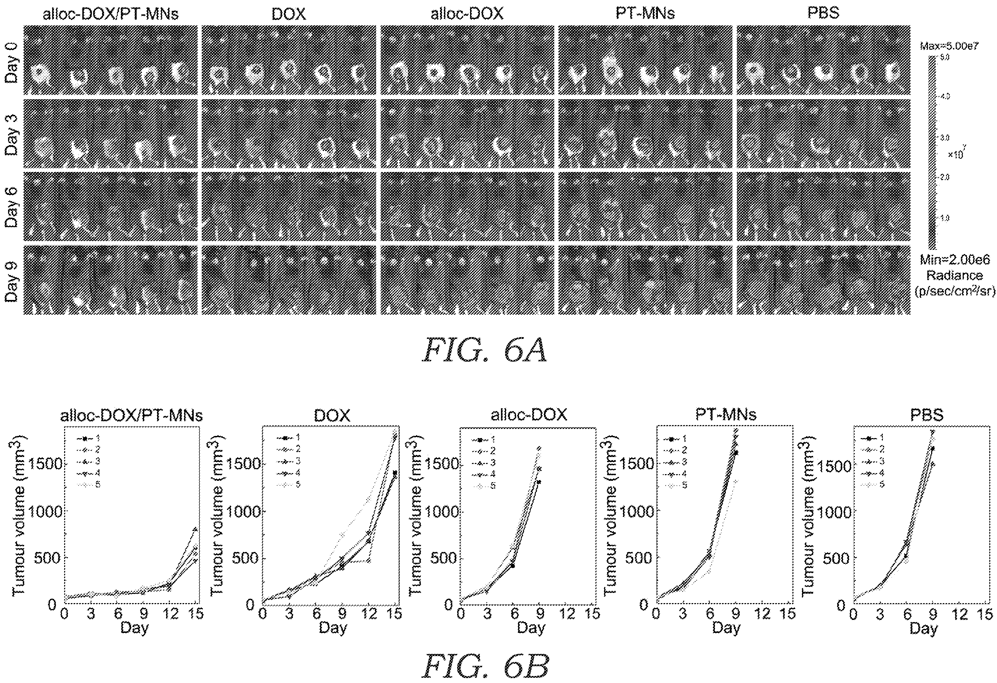

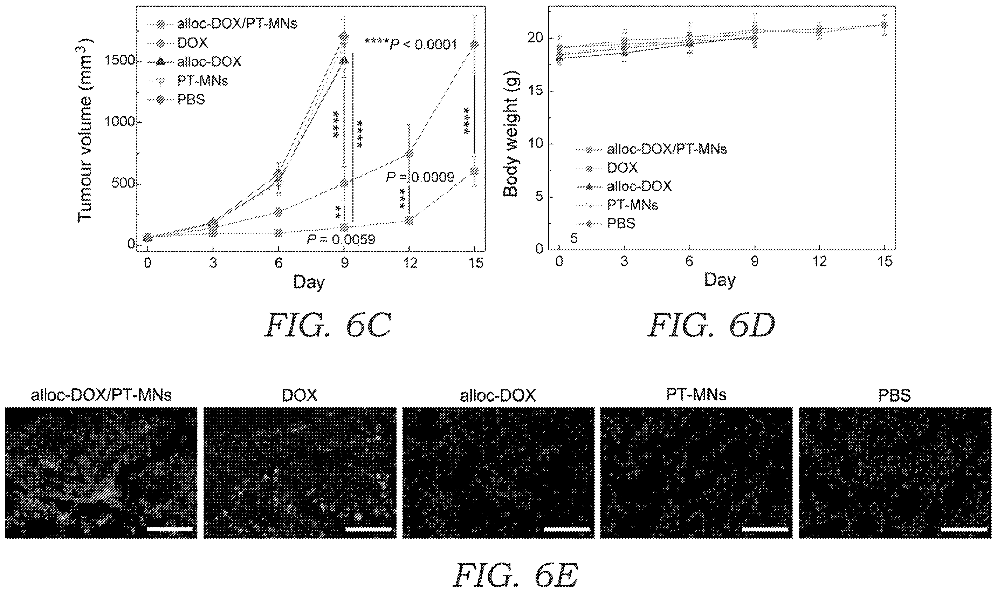

FIGS. 6A-6E illustrate the results of PT-MNs-mediated prodrug activation *in vivo* for anticancer studies. FIG. 6A illustrates bioluminescence imaging of the B16F10 tumor growth in mice treated with alloc-DOX/PT-MNs combination, DOX, alloc-DOX, PT-MNs and PBS. FIG. 6B shows individual tumor growth kinetics of mice in each group. FIG. 6C illustrates changes in the average tumor sizes of each group. Data points represent mean ± s.d. (*n* = 5). ***P* < 0.01, ****P* < 0.001. FIG. 6D is a graph showing changes in the average body weight of each group. Data points represent mean ± s.d. (*n* = 5). FIG. 6E illustrates analysis of cell death mechanism in the tumor tissues after different treatments. The tumor sections were stained with TUNEL-BrdU-Red (green) for apoptosis and Hoechst 33342 for the nuclei (blue). Scale bar, 100 µm.
FIGS. 7A-7G illustrate drug and prodrug concentrations versus time and Pd content in tissues after different treatment. The concentrations of DOX and alloc-DOX in (FIG. 7A) plasma, (FIG. 7B) heart, (FIG. 7C) liver, (FIG. 7D) spleen, (FIG. 7E) lung, (FIG. 7F) kidney and (FIG. 7G) tumor after i.p. injection alloc-DOX with pre-insertion of PT-MNs (alloc-DOX/PT-MNs), i.p. injection of alloc-DOX alone and DOX alone. Data points represent mean ± s.d. (*n* = 5). ***P* < 0.01.
FIG. 7H illustrates a histogram of the content of Pd in plasma (µg/mL) and major organs (µg/g) after application of the PT-MNs for bioorthogonal prodrug activation *in vivo.* Microneedle patches incorporated with Pd nanoparticles (PdNP-MNs) were applied as a positive control for studying Pd leakage. Data points represent mean ± s.d. (*n =* 5).
FIG. 8 illustrates X-ray diffraction (XRD) pattern of the TiO₂ nanosheets, consistent with the standard pattern of TiO₂(B) (JCPDS card no. 74-1940). The intense reflection at 2θ = 7.5° was ascribed to the lamellar structure with layer spacing of ~1.2 nm.
FIG. 9 illustrates UV-Vis absorption spectra of the obtained TiO₂(B) nanosheets. The strong absorption of TiO₂(B) nanosheets in the UV range were then leveraged for photodepositing palladium nanoparticles.
FIG. 10 illustrates Fourier-transform infrared spectra of TiO₂(B) nanosheets (TNSs) and palladium-deposited TiO₂(B) nanosheets (Pd-TNSs). The characteristic absorption bands of ethylene glycol including -CH2 vibration at 2938 and 2870 cm⁻¹, O-H bending vibration at 1630 cm⁻¹ and C-O stretching vibration at 1080 cm⁻¹ were present in both samples.
FIG. 11 illustrates thermogravimetric analysis (TGA) of the TNSs before and after photodeposition of Pd nanoparticles. The ethylene glycol content for TNSs and Pd-TNSs were measured to be 20.13% and 19.58%, respectively, implying that almost no ethylene glycol was lost during the growth of Pd nanoparticles.
FIG. 12 illustrates energy dispersive X-ray spectroscopy of the Pd-TNSs, corresponding to the elemental mapping analysis shown in FIG. 2C.
FIG. 13 illustrates X-ray absorption near edge structure of Pd K-edge for Pd-TNSs with reference materials Pd foil and PdO powder.
FIG. 14 illustrates the synthesis of *bis*-*N*,*N*'-allyloxycarbonyl-caged rhodamine 110.
FIG. 15 illustrates the ¹H NMR (300 M, in DMSO-*d*⁶) of *bis-N,N'-*allyloxycarbonyl-caged rhodamine 110.
FIG. 16 illustrates the standard curve showing the fluorescence intensity at 530 nm (Eₓ = 488 nm, Eₘ = 530 nm) as a function of RhB 110 concentration. Data points represent mean ± s.d. (*n* = 3).
FIGS. 17A and 17B illustrate (FIG. 17A) catalytic conversion of alloc-RhB 110 (100 µM) by sub-stoichiometric amounts of Pd (0.25 mg/mL Pd-TNSs, [Pd]~48.8 µM). Data points represent mean ± s.d. (*n* = 3). FIG. 17B illustrates heterogeneous catalysis study. Data points represent mean ± s.d. (*n* = 3). Pd-TNSs were dispersed in 1 mL PBS buffer for 24 h first and then the solid part was removed by centrifugation. After that, alloc-RhB 110 was added to the upper supernatant, the resulting solution was stirred at 37 °C for 24 hours and analyzed by fluorescence spectrometer.
FIG. 18 illustrates the distribution of PVA, TiO₂ and Pd inside the microneedle (upper left image) by time-of-flight secondary ion mass spectrometry (TOF-SIMS). Signals were collected by the ions of C₃H₃O⁺, TiO⁺ and Pd⁺. Each image was collected with an area of 500 × 500 µm.
FIG. 19 illustrates the mechanical behavior of microneedles made of PVA and Pd-TNSs incorporated PVA. The fracture force of PT-MNs gave a value of 2.97 N/needle, which was about 2-fold larger than that of pure PVA microneedles (1.29 N/needle).

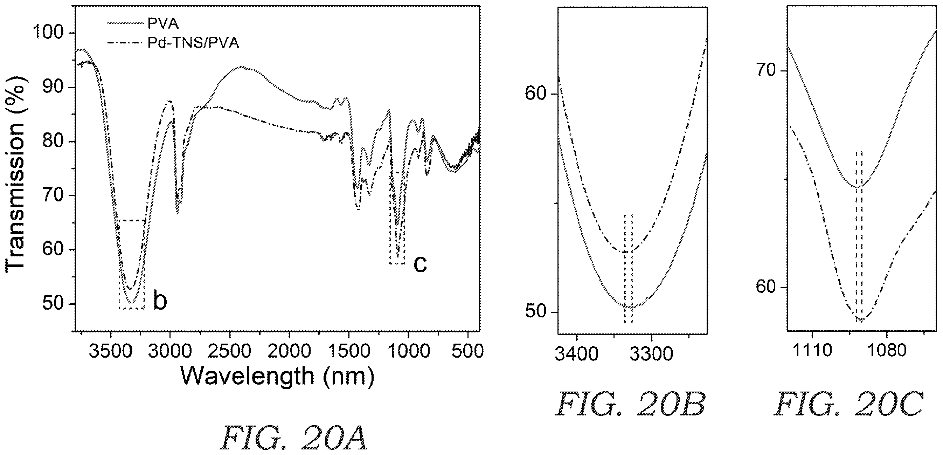

FIGS. 20A-20C illustrate Fourier-transform infrared spectra of PVA and Pd-TNSs-incorporated PVA (FIG. 20A). From the spectra (FIG. 20B), the peak of OH stretching at 3329 cm⁻¹ in pure PVA was shifted to 3333 cm⁻¹ in the Pd-TNSs/PVA nanocomposites. As the O-H stretching shifts to lower wavenumber when the strength of hydrogen bonding increases, the shift towards higher wavenumber in the nanocomposites could be due to the formation of weaker hydrogen bonds between PVA and the ethylene glycols anchoring on the TNSs surface via monodentate mode. Also, the peak attributed to C-O stretching on PVA backbone was shifted from 1092 cm⁻¹ in pure PVA to about 1089 cm⁻¹ in the nanocomposites (FIG. 20C). This change might be caused by the displacement of the TNSs surface-attached ethylene glycol with PVA, which led to the formation of a new interfacial chemical bond Ti-O-C in the nanocomposites. These interfacial interactions between Pd-TNSs with PVA conferred the nanocomposites with higher mechanical property and good stability.
FIG. 21A schematically illustrates the chamber utilized to host microneedle array patches.
FIG. 21B illustrates a photograph of a 3D printed chamber.
FIG. 22A illustrates microscope images of the microneedles at different time points after immersing within water. Scale bar: 200 µm.
FIGS. 22B-22C illustrate the results of the swelling behavior of PT-MNs in PBS buffer. Changes in length (FIG. 22B, ΔL) and width (FIG. 22C, ΔW) of the needles at different time points are shown. The changes were measured around the sites shown in the right top panel of FIG. 22A. After 0.5 h, the swelling of the needles reached a plateau, and the length and width increased about 250 µm and 100 µm, respectively. Also, it could be seen that the microneedles maintained well in aqueous environment. Data points represent mean ± s.d. (*n* = 3).
FIG. 23 illustrates the results showing the amount and percentage of Pd leakage from PT-MNs after catalyzing alloc-RhB 110 activation for 24 h. The total Pd in the microneedles of one PT-MNs patch was about 12 µg. Data points represent mean ± s.d. *(n* = 3). Microneedle arrays patches utilizing free Pd nanoparticles (PdNP-MNs) as nanofillers were utilized as positive control for studying Pd leakage. In comparison with PdNP-MNs, hardly any Pd leakage was detected from PT-MNs.
FIG. 24A illustrates a TEM image of the Pd nanoparticles that used to prepare Pd-doped microneedles; scale bar: 20 nm.
FIG. 24B illustrates a photograph of Pd nanoparticles-doped microneedles.

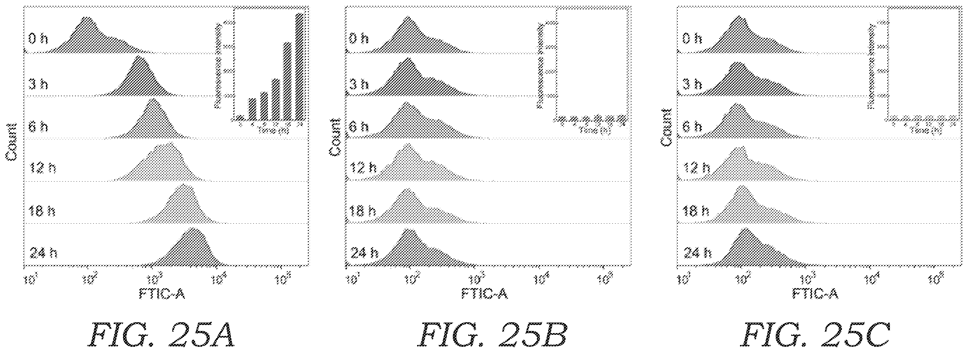

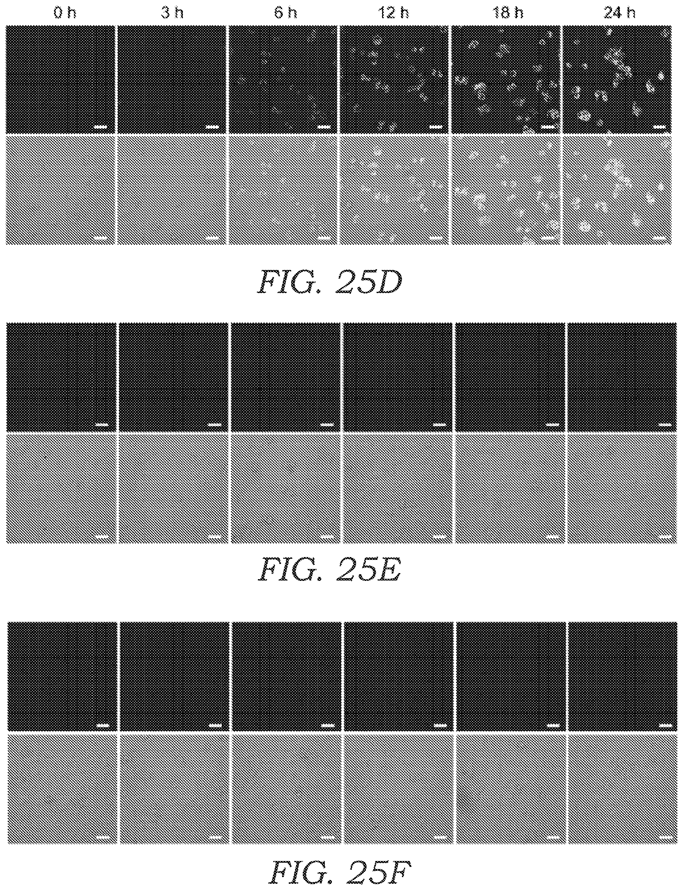

FIGS. 25A-25F illustrate PT-MNs mediated bioorthogonal catalysis in culture of HepG 2 cells. Flow cytometry analysis of the cells after different time treated with alloc-RhB 110 and PT-MNs combination (FIG. 25A), alloc-RhB 110 alone (FIG. 25B) and PT-MNs alone (FIG. 25C). Inset plots show the corresponding fluorescence intensities of the histograms. Confocal laser microscopy images showing the fluorescence changes inside cells after treated with alloc-RhB 110 and PT-MNs combination (FIG. 25D), alloc-RhB 110 alone (FIG. 25E) and PT-MNs alone (FIG. 25F) at different time points (scale bars, 50 µm). Time-dependent increase in fluorescence was directly observed for groups treated with alloc-RhB 110 and PT-MNs combination while no obvious change was detected for control groups, which was consistent with flow cytometry results.

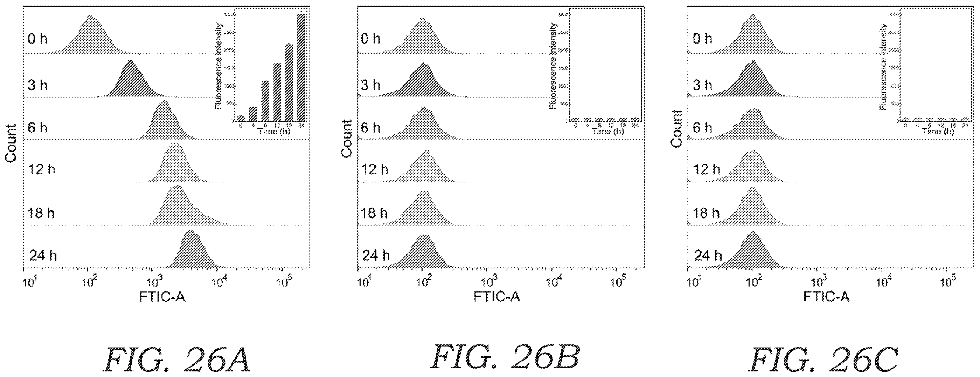

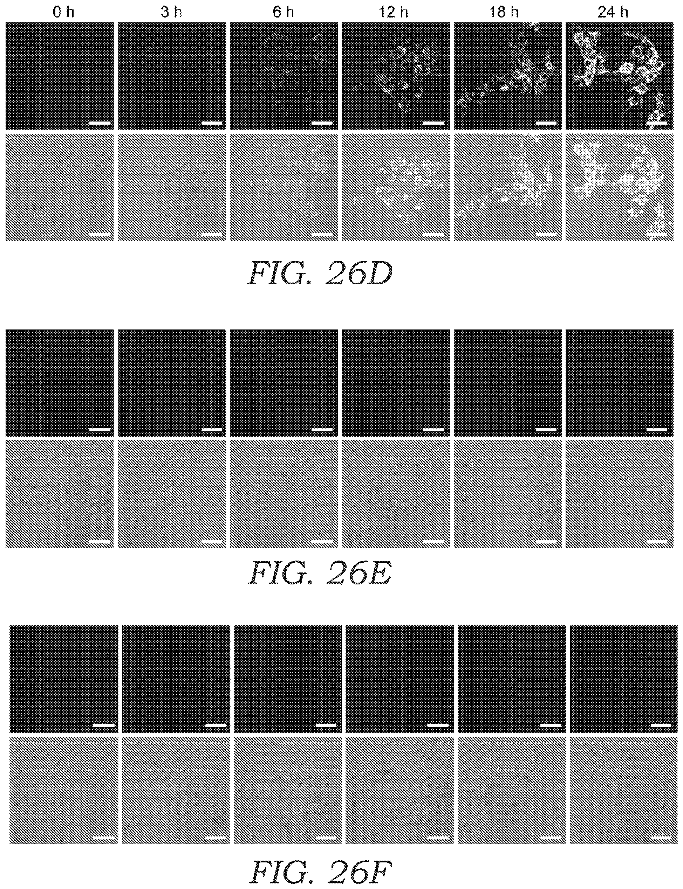

FIGS. 26A-26F illustrates PT-MNs mediated bioorthogonal catalysis in culture of 4T1 cells. Flow cytometry analysis of the cells after different time treated with alloc-RhB 110 and PT-MNs combination (FIG. 26A), alloc-RhB 110 alone (FIG. 26B) and PT-MNs alone (FIG. 26C). Inset plots show the corresponding fluorescence intensities of the histograms. Confocal laser microscopy images showing the fluorescence changes inside cells after treated with alloc-RhB 110 and PT-MNs combination (FIG. 26D), alloc-RhB 110 alone (FIG. 26E) and PT-MNs alone (FIG. 26F) at different time points (scale bars, 50 µm). Time-dependent increase in fluorescence was directly observed for groups treated with alloc-RhB 110 and PT-MNs combination while no obvious change was detected for control groups, which was consistent with flow cytometry results.
FIG. 27 illustrates the synthesis of *N*-allyloxycarbonyl-caged doxorubicin.
FIG. 28 illustrates the ¹H NMR (300 M, in DMSO-*d*⁶) of *N*-allyloxycarbonyl-caged doxorubicin.

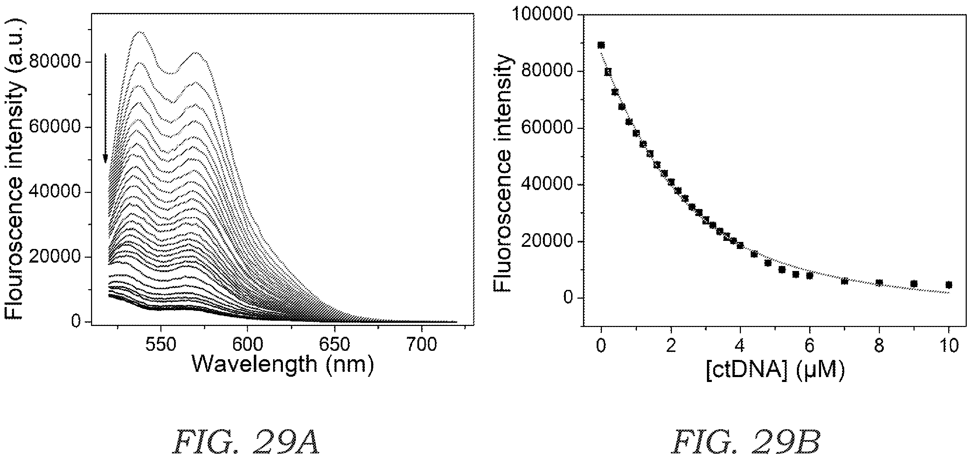

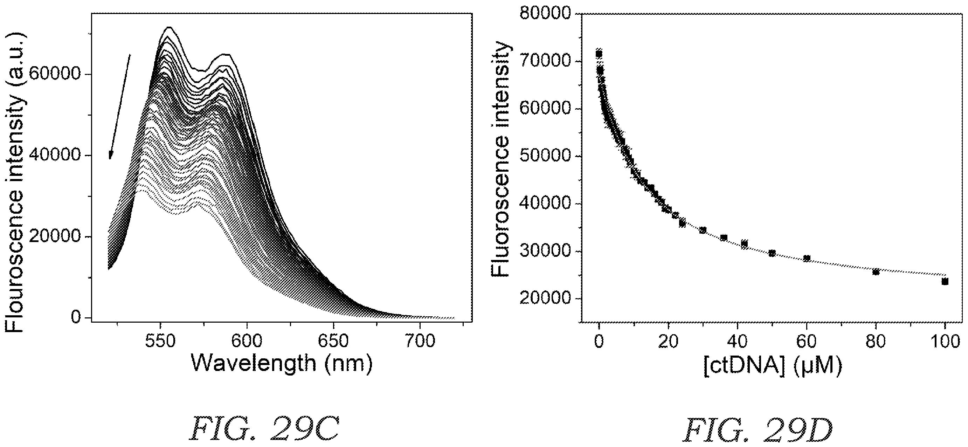

FIGS. 29A-29D illustrate: the spectrofluorometric titrations of ctDNA/DOX (FIG. 29A) and ctDNA/alloc-DOX (FIG. 29C) systems, and the binding isotherms for ctDNA/DOX (FIG. 29B) and ctDNA/alloc-DOX (FIG. 29D) systems. In FIGS. 29B and 29D, F represents the measured fluorescence intensity at each concentration of ctDNA, and F₀ represents the fluorescence of DOX (λ_{Em} = 438 nm) or alloc-DOX (maximum emission) in the absence of ctDNA. Data points represent mean ± s.d. (*n =* 3). DOX is a classical DNA intercalating agent that can insert the planar tetracyclic chromophore between two adjacent base pairs, and meanwhile the electrostatic interaction between the positively charged daunosamine moiety with the DNA phosphate groups can stabilize the intercalation complex. After binding ctDNA, the fluorescence of DOX or alloc-DOX could be quenched, by which one could be measure the binding constant using fluorescence titration method. The binding constants yielded the values K_{ctDNA/DOX} = 9.36× 10⁵ M⁻¹and K_{ctDNA/alloc-DOX} = 7.96× 10⁴ M⁻¹. As the binding constant K_{ctDNA/alloc-DOX} is much lower than K_{ctDNA/DOX}, one can see that the masking of daunosamine moiety with allyloxycarbonyl group effectively weakens the interaction between prodrugs and ctDNA.
FIGS. 30A-30D illustrate the catalytic deprotection of alloc-DOX. FIG. 30A illustrates the reaction mixture without Pd-TNSs (0% yield), (FIG. 30B) reaction mixture with free DOX (theoretic 100% yield), (FIG. 30C) reaction mixture after 24 h (~81.6% yield). FIG. 30D illustrate the catalytic conversion course of alloc-DOX in the presence (curved line) or absence (flat line) of Pd-TNSs. Data points represent mean ± s.d. (*n* = 3).
FIG. 31 illustrates the viability of HepG2 cells treated with DOX, alloc-DOX and alloc-DOX/PT-MNs combination at different drug concentrations. The IC₅₀ of alloc-DOX is about 9.08 µM, which was about 27-times less toxic than DOX (IC₅₀ = 0.34 µM). The combination of alloc-DOX and PT-MNs gave an IC₅₀ of 0.42 µM. Data points represent mean ± s.d. (*n* = 3).
FIG. 32 Viability of 4T1 cells treated with DOX, alloc-DOX and alloc-DOX/PT-MNs combination at different drug concentrations. The IC₅₀ of alloc-DOX is about 8.06 µM, which was around 37-times less toxic than DOX (IC₅₀ = 0.22 µM). The combination of alloc-DOX and PT-MNs gave an IC₅₀ of 0.29 µM. Data points represent mean ± s.d. (*n =* 3).
FIGS. 33A-33B illustrate HPLC analysis of the extracellular culture medium after incubating with (FIG. 33A) alloc-DOX (1 µM) and (FIG. 33B) allox-DOX (1 µM)/PT-MNs combination for 24 h.
FIG. 34 illustrates the viability of B16F10 cells after different time exposure to PT-MNs. No obvious toxicity was triggered by the patch device. Data points represent mean ± s.d. (*n* = 3).
FIG. 35 illustrates the amount of leaked Pd per well after 48 h immersing in cell culture. PdNP-MNs were utilized as a positive control for studying Pd leakage. Compared to PdNP-MNs, no significant Pd leakage from PT-MNs was detected. Data points represent mean ± s.d. (*n* = 3).
FIGS. 36A-36C illustrate skin insertion capacity of the PT-MNs patch. FIG. 36A is a photograph showing the insertion of a piece of PT-MNs patch into the skin over the tumor. FIG. 36B illustrates microholes that were observed after removing the microneedles at the end of each treatment. FIG. 36C shows that the microholes were temporary and could gradually reseal over 2 h after removing the PT-MNs.
FIGS. 37A-37B illustrate (FIG. 37A) Representative SEM image of the PT-MNs after removing from mice and lyophilization. Scale bar: 200 µm. (FIG. 37B) Close inspection of the surface of the PT-MNs revealed porous structures, which could be due to the swelling of PT-MNs in the presence of body fluid after insertion. Scale bar: 2 µm.
FIGS. 38A-38B illustrates in vivo toxicity profiles of DOX (FIG. 38A) and alloc-DOX (FIG. 38B) after intraperitoneal (i.p.) injection every 3 days. When the dose of DOX was over 10 mg/kg, serious body weight loss over time and death (15 mg/kg at the seventh day and 10 mg/kg at the fourteenth day) were observed. For mice treated with alloc-DOX, the body weight changes remained within 10 % even the dose was given at 150 mg/kg. These results suggested the low toxicity of the prodrug alloc-DOX. Data points represent mean ± s.d. (*n* = 5).
FIG. 39 illustrates Hematoxylin and eosin-stained sections of the major organs collected from mice at the end of different treatments including alloc-DOX/PT-MNs combination, DOX, alloc-DOX, PT-MNs and PBS. Scale bar for all panels: 200 µm.
FIGS. 40A-40D illustrates serum levels of IFN-y (FIG. 40A), IL-12 (FIG. 40B), IL-6 (FIG. 40C) and TNF-*α* (FIG. 40D) in melanoma-bearing mice after treated with alloc-DOX/PT-MNs combination, DOX, alloc-DOX, PT-MNs and PBS. Data points represent mean ± s.d. (*n* = 5).

### Detailed Description of Illustrated Embodiments

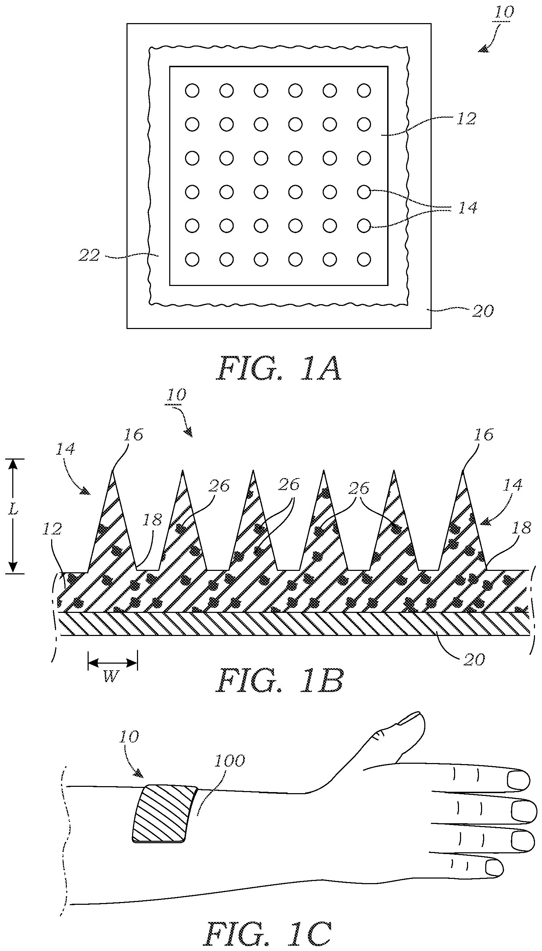

FIG. 1A illustrates plan view of a patch 10 (also referred to herein as PT-MNs 10) that may be used for *in vivo* biorthogonal catalysis applications in mammalian tissue 100 (FIG. 1C). The patch 10 includes a base or substrate 12 that includes a plurality of microneedles 14 that extend or project from the substrate 12. The patch 10 may in some embodiments be partly or entirely biodegradable. However, in other preferred embodiments, the patch 10 is not made to be biodegradable and is instead removed from the site of application on the tissue 100. Removal of the patch 10 ensures that any potentially hazardous or potentially toxic transition metals in the patch 10 are not present at the site of application. The term biodegradable in the context of a biodegradable patch 10 refers to the base or substrate 12 and/or the microneedles 14 being formed from a material that is biodegradable. The plurality of microneedles 14 generally extend or project in a perpendicular direction from a surface of the base or substrate 12. The plurality of microneedles 14 may be arranged in a regular repeating array as illustrated in FIG. 1A or, alternatively, they may be arranged in a random pattern or array of microneedles 14. In one embodiment, the plurality of microneedles 14 that are formed on the base or substrate 12 may have substantially similar shapes and sizes. However, in other embodiments, the plurality of microneedles 14 may have different shapes and/or sizes. For example, the perimeter region of the array or field of microneedles 14 that extend from the base or substrate 12 may be longer or have different shapes than those in the central region of the patch 10 to better secure the patch 10 to site of application.

In one particular embodiment, the microneedles 14, as their name implies, have a needle-like shape. For example, the microneedles 14 may include a sharpened tip 16 (seen in FIG. 1B) that aid in penetrating the epidermal layer of the skin tissue 100 (seen in FIG. 1C), although the tip 16 may not perfectly sharp. For example, the tip 16 may have small radius (e.g., around 10 µm) but still provide enough sharpness for tissue penetration. The patch 10 may also be used with other types of tissue 100 beyond skin. This may include other organ tissues 100 beyond skin. The tissue 100 may be healthy in some embodiments while in other embodiments the tissue 100 may be diseased. The length (L) of the microneedles 14 may vary although typically the microneedles 14 extend less than about 2 mm from the base or substrate 12 (FIG. 1B). A typical length of the microneedles 14 is about 300 µm to about 2,000 µm, although the dimensions may extend outside this range. In experiments described herein, the height of the microneedles 14 was 1,500 µm. The base 18 of the microneedle 14 is wider than the tip 16. Typically, the base 18 of the microneedle 14 may have a diameter or width (W) that is less than about 700 µm (e.g., 500 µm base and a height of around 1,500 µm) (FIG. 1B). The particular dimensions and shape(s) of the microneedles 14 are controlled by the particular construction of the mold that is used to form the patch 10, which is described more in detail below. The spacing or pitch of the microneedles 14 may also vary. In the experiments described herein, an array of 15 × 15 microneedles 14 were formed with 800 µm center-to-center spacing. It should be appreciated that a wide variety of arrangements of microneedles 14 and spacing may be used in connection with the patches 10 described herein.

Still referring to FIGS. 1A and 1B, the base or substrate 12 which holds the microneedles 14 may be optionally bonded or otherwise adhered to a backing material 20 (e.g., through the use of an adhesive, chemical linking, or the like). The backing material 20 may be made from a woven fabric, a plastic material such as polyvinylchloride, polyethylene, or polyurethane, or latex. The backing material 20 may be flexible so that the patch 10, when applied, can conformally cover the tissue 100 (seen in FIG. 1C). Optionally, the backing material 20 may include an adhesive material 22 that covers all or a portion of the tissuefacing surface of the backing material 20. For example, adhesive may be formed on the backing material 20 around the periphery of the base or substrate 12 or the backing material 20 so that the base or substrate 12 may be secured in place to the surface of the tissue 100. The adhesive material 22 aids in securing the patch 10 to the tissue 100. The adhesive material 22 may include resins (e.g., vinyl resins), acrylates such as methacrylates epoxy diacrylates. Of course, in other embodiments, the patch 10 may not have a backing material 20.

The base or substrate 12 and the microneedles 14 may be relatively rigid in a substantially dry state. Because of this, in one alternative embodiment, multiple sub-patches may be integrated into the backing material 20 to make the final patch 10. This may be useful for large coverage areas or curved surfaces that may pose a risk of breakage to the base or substrate 12. The various sub-patches, while generally rigid, are still able to conform to the surface of the tissue 100 (e.g., FIG. 1C) due the flexible backing material 20 which enables bending of the overall patch 10. Because individual sub-patches are smaller in size these do not experience significant bending stresses which would otherwise cause a larger, more rigid structure to break in response to bending and/or manipulation. Bending or flexing can occur within the backing material 20 between the locations of where the sub-patches are located (e.g., between the rows and columns of sub-patches). While the patch 10 may be in a substantially dry state upon application to tissue 100, the patch 10 including the microneedles 14 will become hydrated and swell in response to exposure to the aqueous fluid of the tissue 10. In addition, by being in a dry state at the time of application, the microneedles 14 can more easily penetrate into the tissue 10.

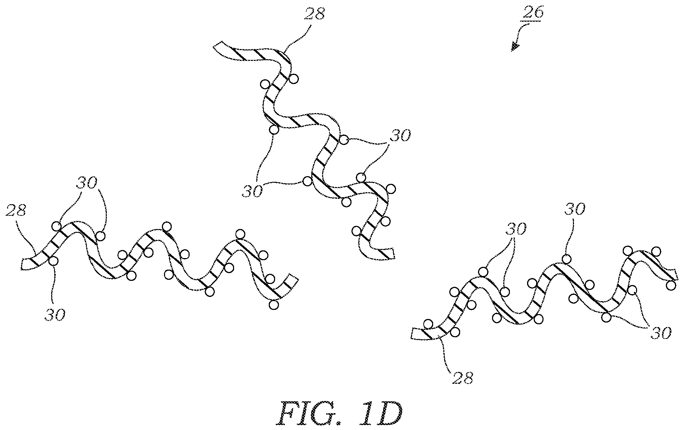

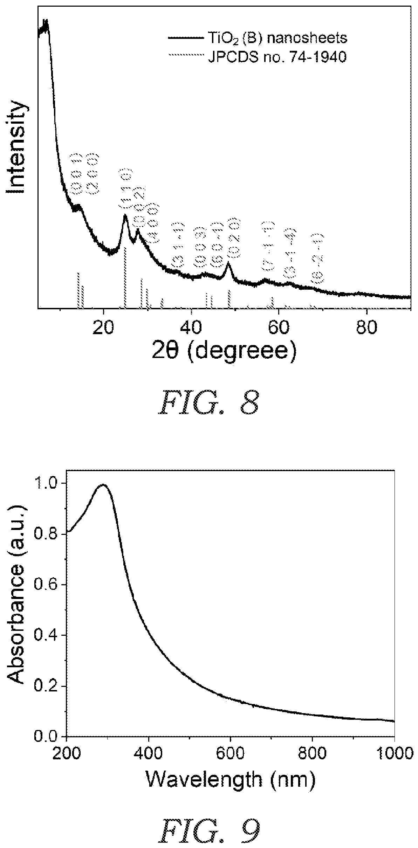

The microneedles 14 may have a number of different shapes and configurations including, for example, a pyramid, cone, cylindrical, tapered tip, canonical, square base, pentagonal-base canonical tip, side-open single lumen, double lumen, and side-open double lumen. The plurality of microneedles 14 swell upon breaching or penetrating the biological barrier and absorbing fluid from the surrounding tissue 100. The microneedles 14 may swell from about 100% to about 300% (wt. basis). The microneedles 14 swell and, in one embodiment, form a flexible material. In some embodiments, the microneedles 14 are also biodegradable and dissolve over time. In one embodiment, with reference to FIG. 1B, the plurality of microneedles 14 are formed from polyvinyl alcohol (PVA) matrix with TiO₂ nanosheet-supported palladium nanoparticles (Pd-TNSs) 26 used as a nanofillers. With reference to FIG. 1D, the nanofiller 26, in one preferred embodiment, includes thin TiO₂ nanosheets (TNSs) 28 that have one or more surfaces populated with metallic nanoparticles 30. FIG. 8 illustrates X-ray diffraction (XRD) pattern of the TiO₂ nanosheets. The intense reflection at 2θ = 7.5° was ascribed to the lamellar structure with layer spacing of ~1.2 nm. FIG. 9 illustrates UV-Vis absorption spectra of the obtained TiO₂(B) nanosheets. The strong absorption of TiO₂(B) nanosheets in the UV range were then leveraged for photodepositing palladium nanoparticles 30.

The metallic nanoparticles 30 function as a catalyst. While palladium (Pd) nanoparticles 30 are the focus of one particular embodiment, it should be understood that other metal-based nanoparticles 30 may also be used. These include, other transition metal catalysis and alloys and/or metal complexes of the same (e.g., gold (Au), ruthenium (Ru), iridium (Ir), or copper (Cu)). The base or substrate 12 of the patch may also be made from the same or different materials as the microneedles 14. The PVA serves as a matrix material and entraps Pd-TNSs 26 within the pore walls of the matrix material.

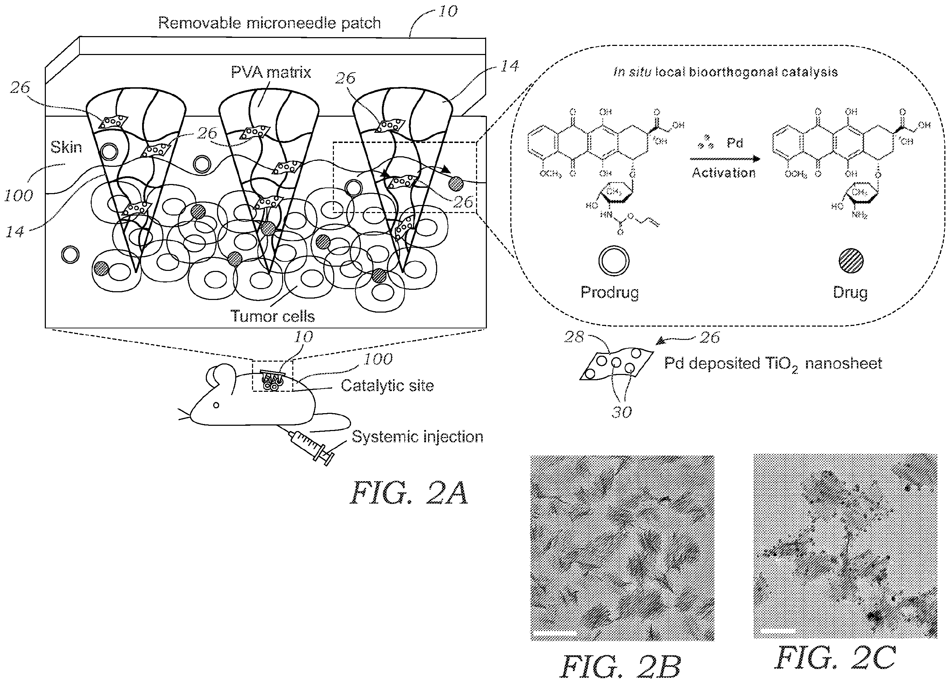

To use the patch 10, in one embodiment and with reference to FIG. 2A, the patch 10 is applied to tissue 100 by applying gentle force to insert the microneedles 14 into the tissue 100. The microneedles 14 penetrate the tissue 100 upon application of the patch 10. The patch 10 may be applied to the target tissue 100 to be treated. This may include diseased tissue 100 in one embodiment (e.g., cancerous tissue as one example). In another embodiment, the patch is applied to healthy tissue 100. The patch 10 may also be applied to a location that is remote from the tissue 100 to be treated. For example, the patch 10 may be applied to skin tissue 100 even though the desired target tissue 100 to be treated by the therapeutic agent or drug may is not the skin tissue 100. An optional adhesive may also be used to secure the patch 10 the tissue 100. The microneedles 14 are then exposed to a prodrug by systemic and/or local delivery (e.g., injection such as intraperitoneally). The patch 10 is preferably delivered prior to administration of the prodrug and some elapsed time may be needed (e.g., tens of minutes to an hour or more) to allow for swelling of the microneedles 14. The prodrug that is administered to the mammal is then catalyzed by the Pd-TNSs 26 in the patch 10 into a therapeutic agent or drug which then leaves the microneedles 14 into the surrounding tissue 100. This is schematically illustrated in FIG. 2A. In one embodiment, the prodrug is N-(allyloxycarbonyl)doxorubicin (alloc-DOX), it should be appreciated that other allyloxycarbonyl-modified prodrugs may also be used in a similar manner. In addition, other prodrugs with good water solubility could be delivered, for example, intravenously. However, the prodrug alloc-DOX is too hydrophobic and was thus injected intraperitoneally.

### Experimental

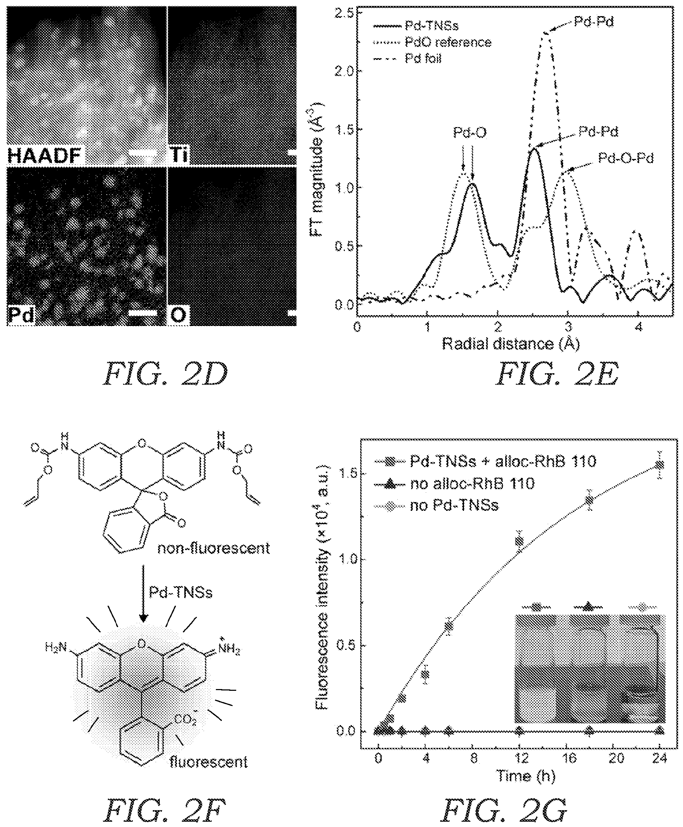

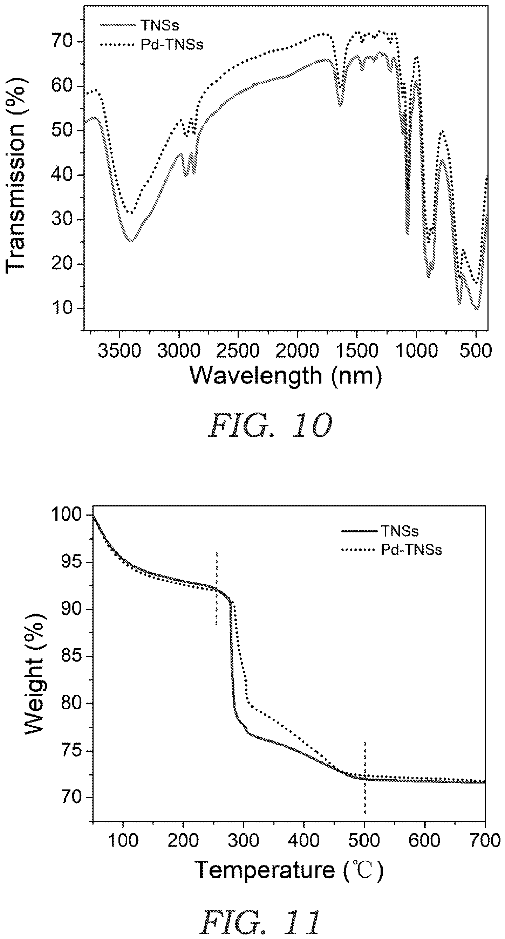

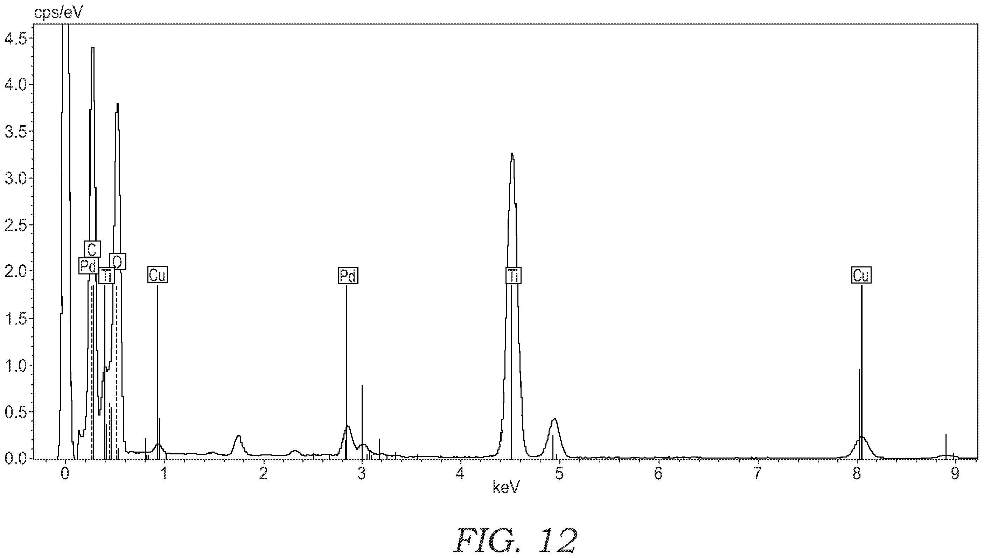

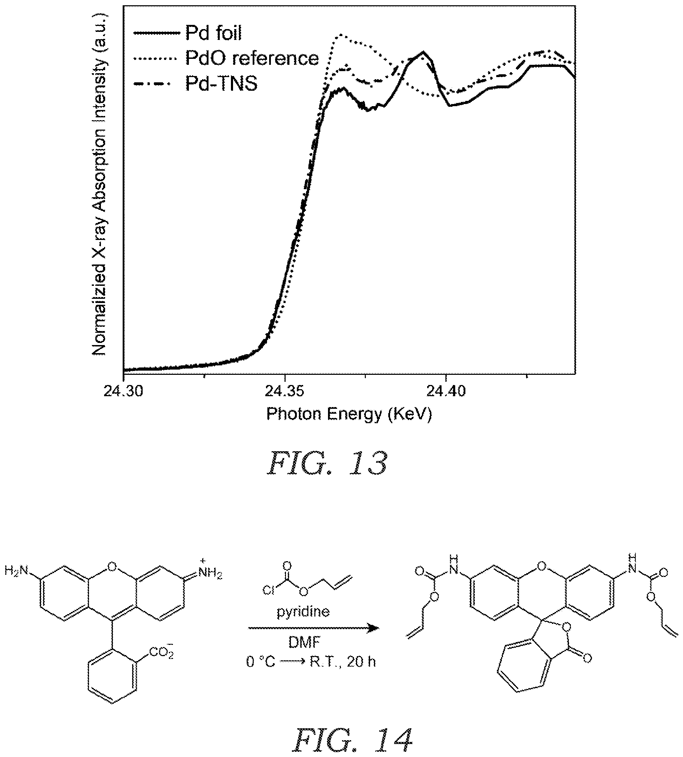

**Synthesis of Pd-TiO₂ Nanofillers.** Ultrathin TiO₂ nanosheets (TNSs) 28 were prepared by solvothermal hydrolysis of TiCl₄ in ethylene glycol and utilized as the support for growth of Pd catalysts (FIGS. 8-9). FIG. 2B shows a representative transmission electron microscopy (TEM) image of the obtained TNSs 28 and they were wrinkled due to the surface tension, which is often observed for two-dimensional materials like graphene. As indicated by infrared spectroscopy and thermalgravimetric analysis, the TNSs 28 were covered with ethylene glycol (~20 w.t.%, FIGS. 10-11). By a photochemical process, Pd nanoparticles 30 with average size ~8 nm were deposited over the surface of the TNSs 28 (Pd-TNSs, FIG. 2C), where the distribution of Pd, Ti and O in the nanocomposites was revealed by elemental mapping analysis (FIG. 2D, FIG. 12) and the Pd content was determined to be about 2.1 w.t.% by inductively coupled plasma mass spectrometry (ICP-MS). Notably, no obvious change was observed in either the morphology of the nanosheets 28 or the amount of surface-attached ethylene glycol (FIG. 10) after the deposition of Pd. Such two-dimensional structures with high exposed Pd surface would be favorable for mass transfer during catalysis. Furthermore, the chemical state and short-range structure of Pd in the Pd-TNSs hybrid nanofiller 26 was studied by X-ray absorption near-edge structure (XAENS) and extended X-ray absorption fine structure spectra (EXAFS) at Pd K-edge, respectively. Compared with PdO, the XANES of Pd-TNSs showed closer edge position to Pd foil, indicating dominant form of metallic Pd (FIG. 13). This is further confirmed by the existence of strong Pd-Pd bonding in Pd-TNSs, as-revealed by Fourier transformed-EXAFS (FIG. 2E). However, different from the Pd foil, Pd-O bonding in the region of 1.5 to 2 Å was also observed in Pd-TNSs 26, which suggested that the photo-reduced Pd nanoparticles 30 anchored onto the TNSs support mainly via Pd-O bonds.

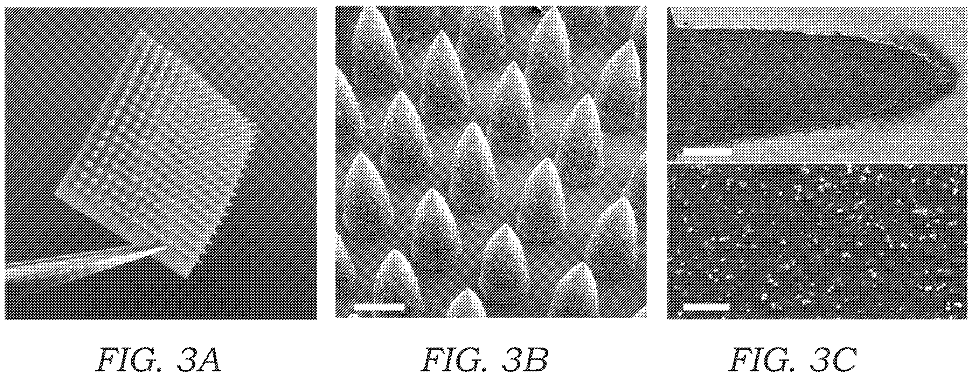

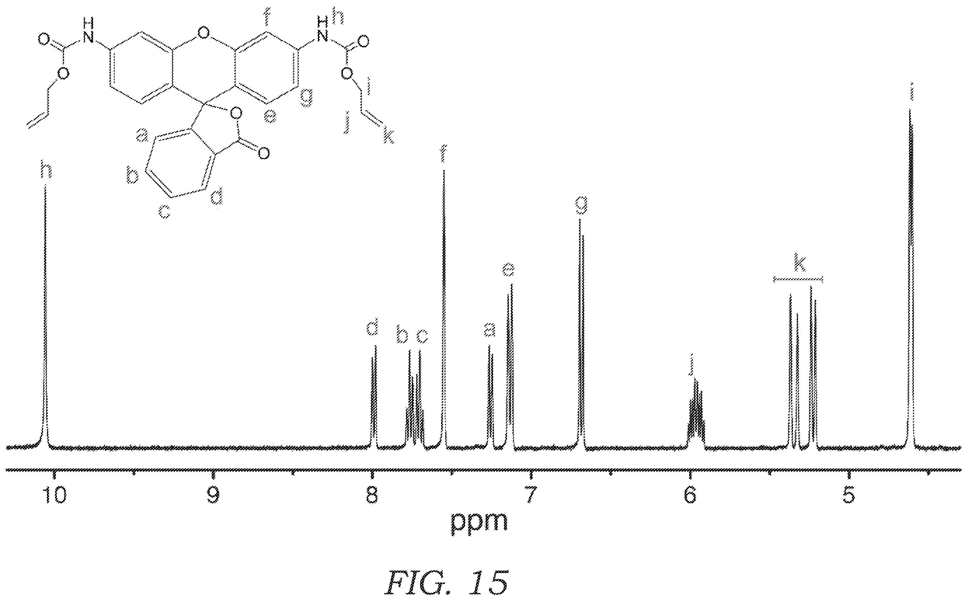

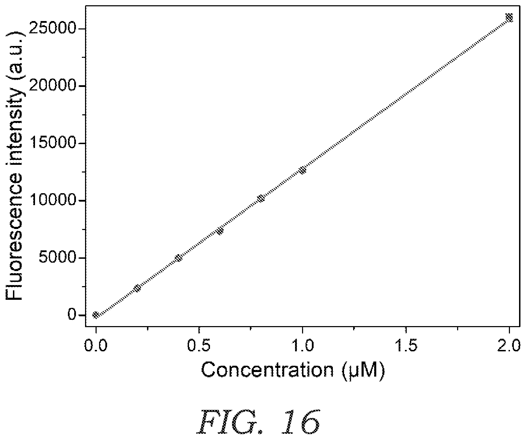

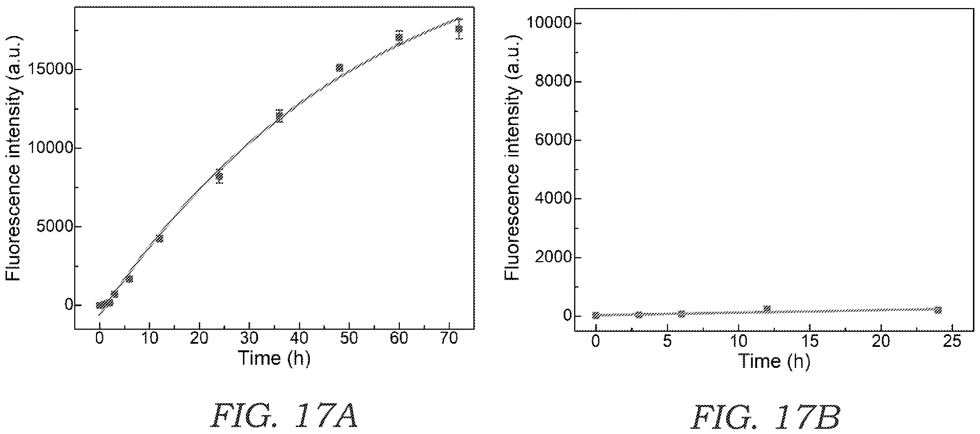

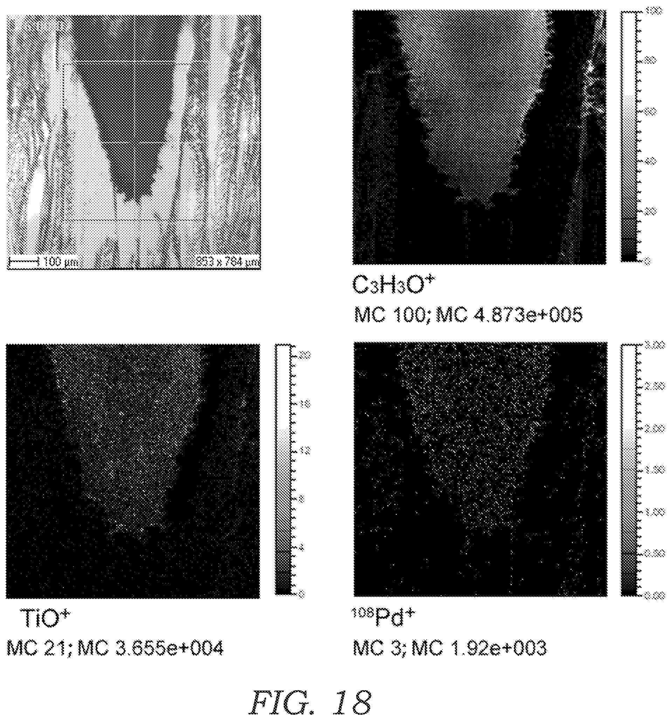

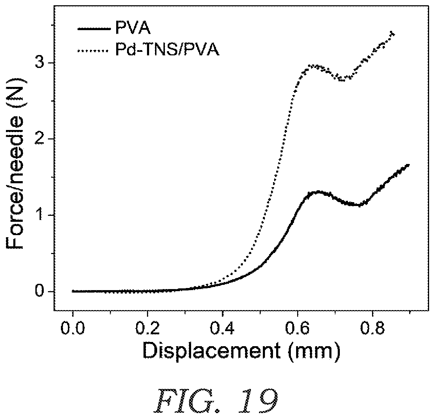

The performance of Pd-TNSs 26 for mediating bioorthogonal catalysis was examined using *bis*-*N*,*N*'-allyloxycarbonyl-caged rhodamine 110 (alloc-RhB 110, FIGS. 14-15) as the substrate in an isotonic media, PBS buffer (pH 7.4). As shown in FIGS. 2F, 2G upon rhodamine 110 (RhB 110) was released by catalytic cleavage of the caging group, green fluorescence gradually increased for solutions containing both Pd-and substrate. The conversion was determined to be 48.8% by using the standard curve (FIG. 16). By contrast, no generation of fluorescence was detected for control groups with either catalysts or substrate omitted over the same time. The catalytic nature of the reaction was confirmed by using sub-stoichiometric amounts of Pd, although a longer time, 60 h, was required to reach the similar conversion efficiency (FIG. 17A). Also, to demonstrate that the conversion was directed by heterogenous catalysis, the Pd-TNS 26 were dispersed in PBS buffer for 24 h first and then the suspension was centrifugated. Afterwards, the upper supernatant was mixed with alloc-RhB 110 and incubated for 24 h, which failed to give detectable fluorescent RhB 110 (FIG. 17B).
**Fabrication of Catalytically Active Microneedle Patches.** To make a device for mediating bioorthogonal catalysis, microneedle array patches 10 made of PVA incorporated with Pd-TNS nanofillers 26 (~6.25 wt%) were prepared. Generally, the wherein the Pd-TNS nanofillers 26 (i.e., TiO₂ nanosheet-supported metallic nanoparticles) comprise between about 2 wt% and about 10 wt% of the patch 10. The microneedle-based patch 10 with Pd-TNS nanofillers 26 is also referred to herein as PT-MNs 10. PVA was chosen as the microneedle matrix because of its unique character of forming microcrystalline regions as physical crosslinkers, non-toxic nature and good compatibility with metal oxide particles. The microneedles 14 were arranged in 15×15 array (FIG. 3A), and each one was conical in shape with a tip radius of 10 µm, a height of 1500 µm, a base diameter of 500 µm and a center-to-center space of 800 µm (FIG. 3B). The overall incorporation of Pd-TNS 26 inside the microneedle 14 was revealed by time-of-flight secondary ion mass spectrometry (FIG. 18). Moreover, SEM image of the longitudinal section of one microneedle 14 showed that the Pd-TNS 26 were homogeneously dispersed inside the PVA matrix (FIG. 3C). Measurement of the fracture force of the microneedles 14 gave a value of 2.97 N/needle, which was about 2-fold higher than that of pure PVA microneedles (FIG. 19). This increase could be attributed to the good dispersion of Pd-TNS 26 inside the matrix (FIG. 3C) and the interfacial interactions between TNS 28 and the hydroxyl groups on PVA backbone (FIGS. 20A-20C), which reinforced the strength of PVA composites. Importantly, such enhanced strength facilitates skin insertion.

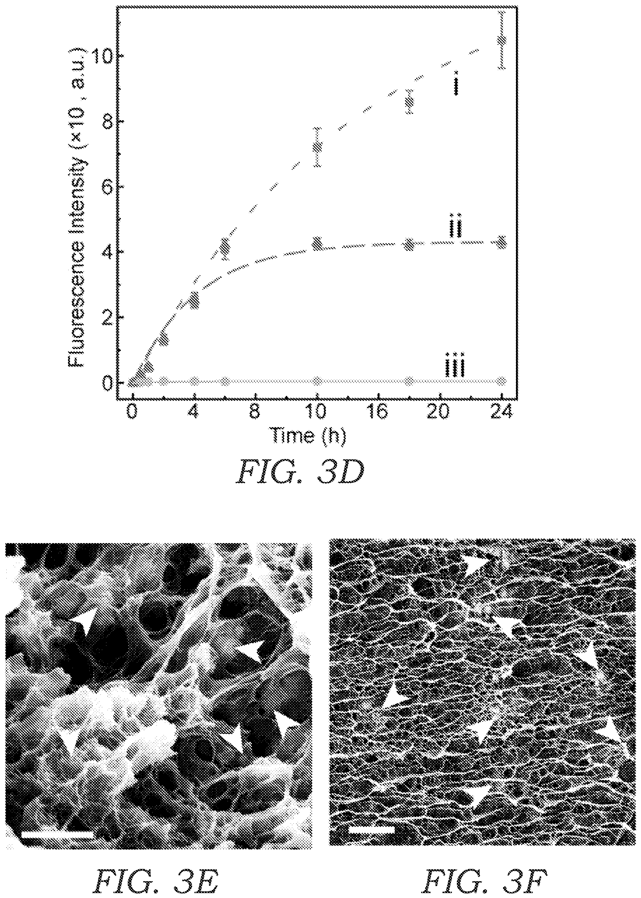

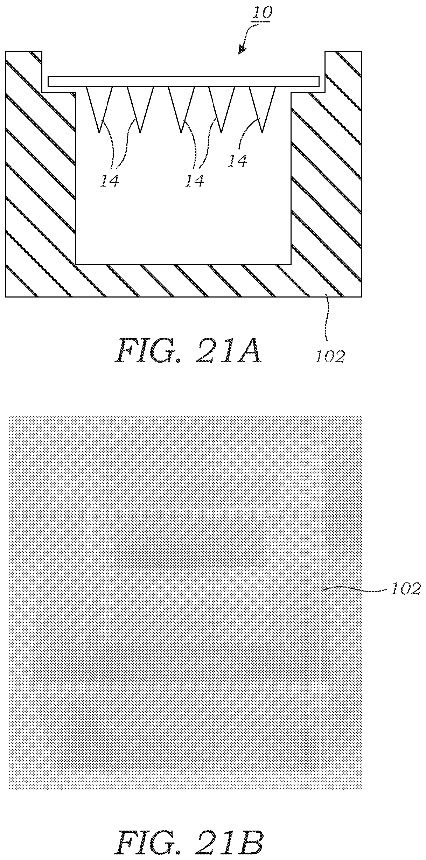

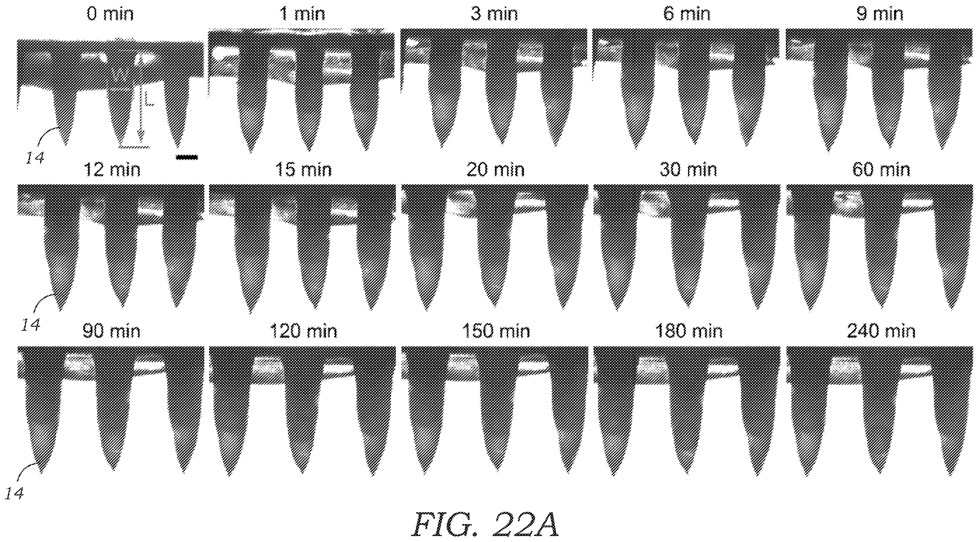

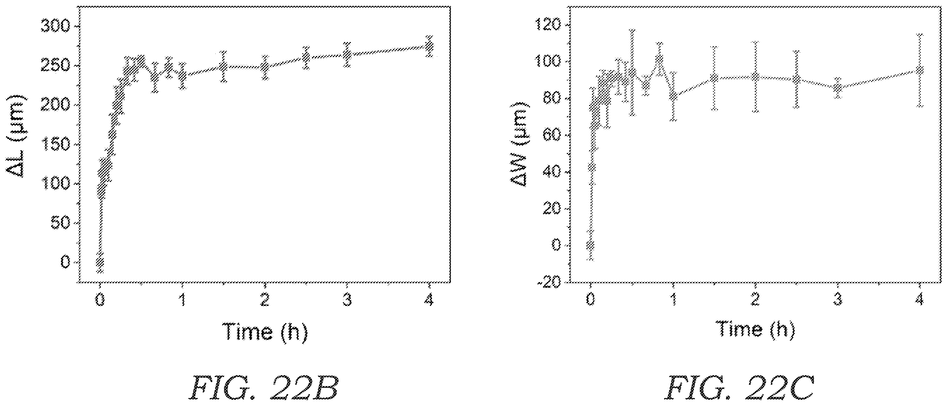

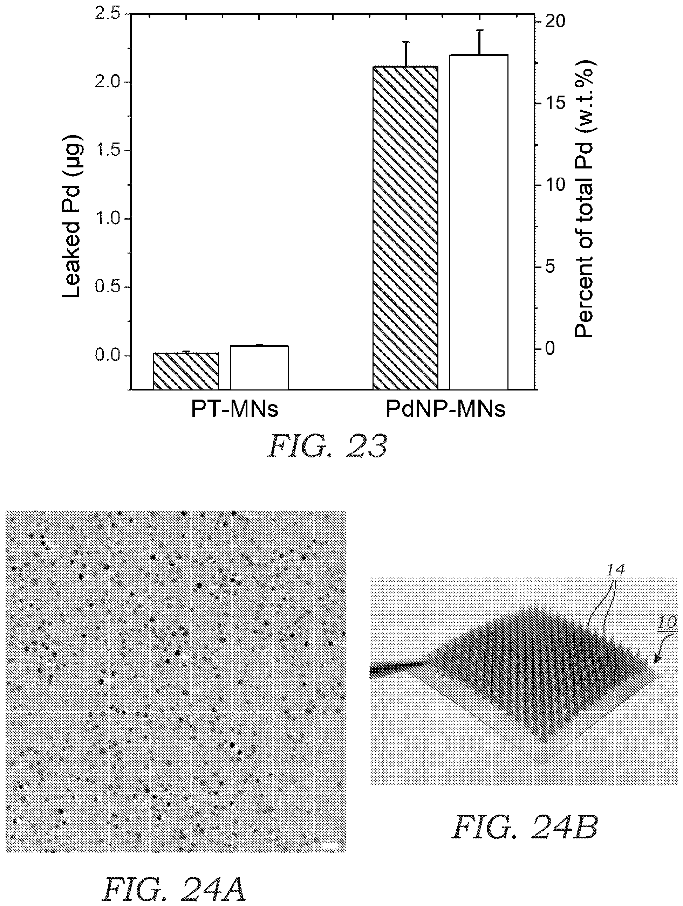

To study the catalytic efficacy of this patch 10, the microneedles 14 were immersed in a homemade chamber 102 as best illustrated in FIG. 21A (also seen in FIG. 21B) filled with alloc-RhB 110-contained PBS buffer solution (FIGS. 3D-i). Like the case of free Pd-TNS studied above, RhB 110 were gradually released with the assistance of PT-MNs 10, whereas no obvious fluorescence was detected for control groups utilizing blank PVA MNs (FIG. 3d-iii). Meanwhile, the swelling behavior of the microneedles 14 were studied by an optical microscope, which revealed that the length and width of the microneedles 14 increased quickly after immersed within water and reached plateaus within 30 min (FIGS. 22A-22C). After further lyophilizing the swelled microneedles 14, three-dimensional network with pores ranging from several to tens of microns were formed both on the surface and inside the needle matrix with Pd-TNSs 26 entrapped within the pore walls (FIG. 3E, 3F). Such hierarchical porous structure is beneficial for facilitating the transport of substrate/product molecules to contact with/leave the catalytic sites and thereby promoting their conversion. At the same time, the Pd content in the aqueous part was measured by ICP-MS, where about 0.24 w.t.% of Pd was leaked during the whole reaction time, suggesting the stability of the patch 10 (FIG. 23). Such stability could be due to the interaction between the Pd and TNSs support and the interaction between TNS 28 and PVA, which together held the Pd catalyst inside the PVA matrix. This conclusion is further supported by two additional experiments. One is to remove the PT-MNs 10 in the midterm, where no further increase in fluorescence intensity was detected (FIG. 3D-ii). The other one showed that about 18.1 w.t.% of Pd was released over the same soaking time when nonsupported free Pd nanoparticles (FIGS. 24A-24B) were used as the putative nanofillers (FIG. 23).

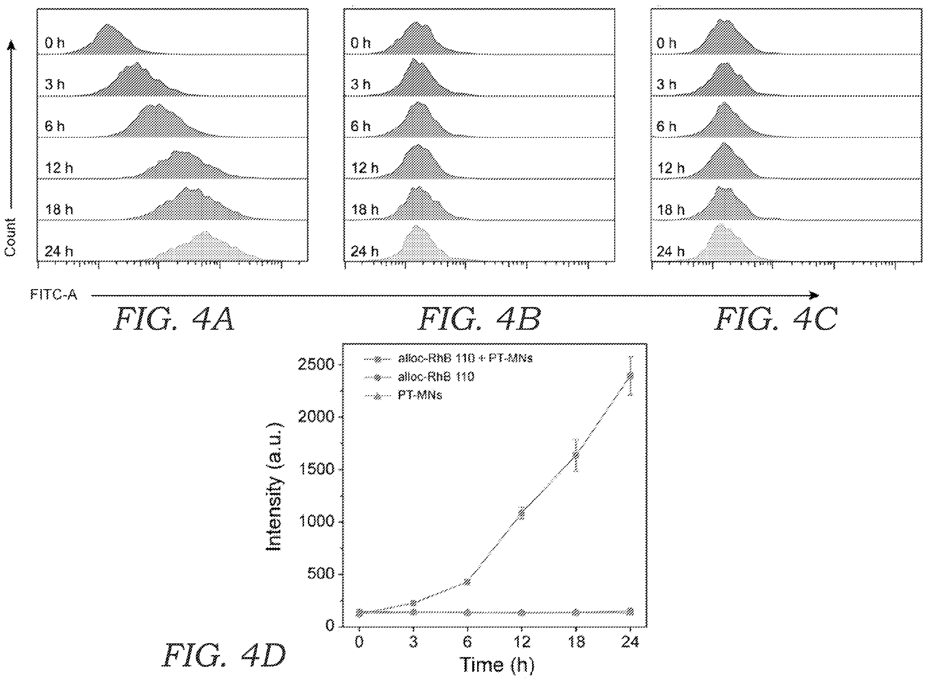

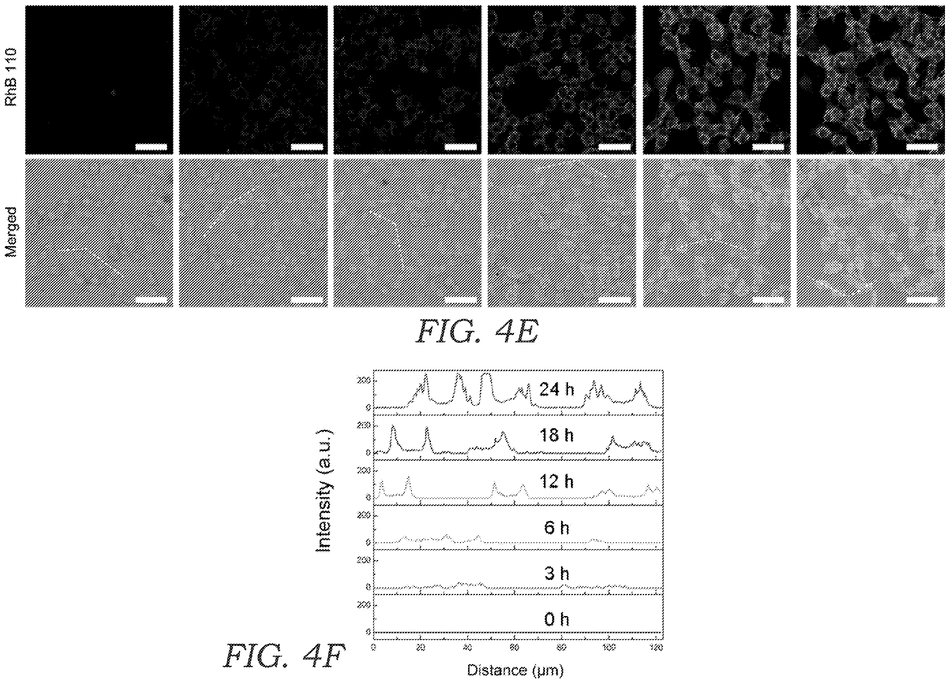

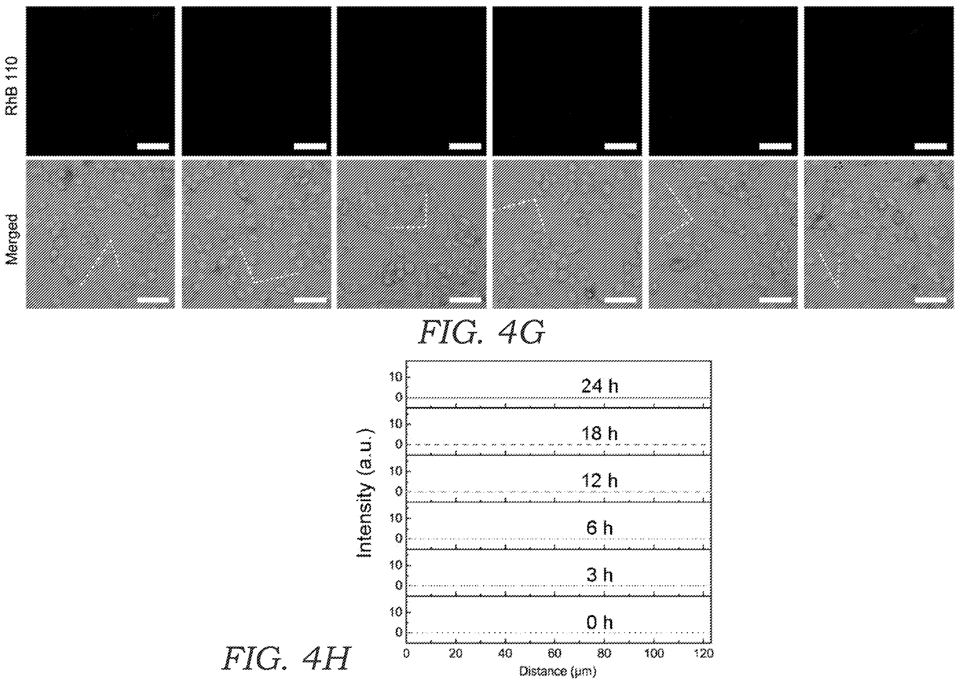

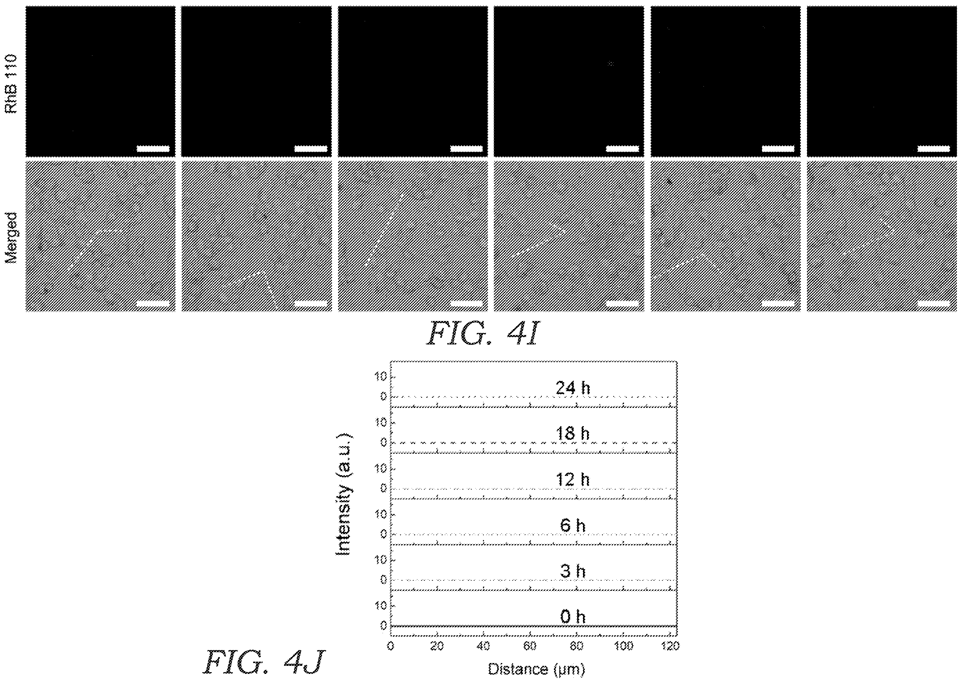

**PT-MNs mediated bioorthogonal catalysis *in vitro.*** Having characterized the catalytic activity of PT-MNs 10 in mediating profluorophore activation in solution, their potency was studied in mediating bioorthogonal catalysis in cellular culture. B16F10 cells were incubated in culture medium containing 25 µM alloc-RhB 110, within which the microneedles 14 of PT-MNs 10 were immersed in the extracellular space. The cells were then analyzed by flow cytometry and confocal microscope at different time points. As indicated by flow cytometry (FIG. 4A), the fluorescence of the whole cell population increased in a time-dependent fashion. While for control groups in the absence of PT-MNs 10 or alloc-RhB 110, no fluorescence evolved over all the time (FIG. 4B, 4C, 4D). Consistent with the flow cytometry results, confocal laser microscopy allowed to visualize the gradually increased green fluorescence inside the cells treated with PT-MNs 10 and alloc-RhB 110 combination (FIG. 4E, 4F), but no distinct fluorescence was seen for the control groups (FIG. 4G-4J). These results demonstrated that PT-MNs 10 could efficiently implement bioorthogonal catalysis *in vitro* to trigger the uncage of alloc-RhB 110. The fluorophores produced in the extracellular medium promoted their diffusion into the entire cell population. In addition, importantly, similar catalytic trend could be replicated with HepG2 (FIGS. 25A-25F) and 4T1 cells (FIGS. 26A-26F), suggesting the generality of the device to mediate bioorthogonal catalysis in different extracellular environments.

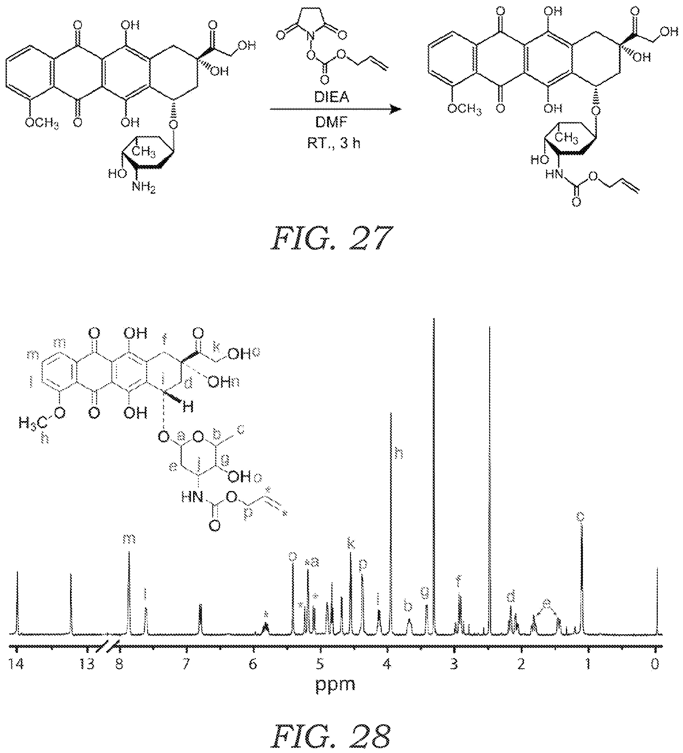

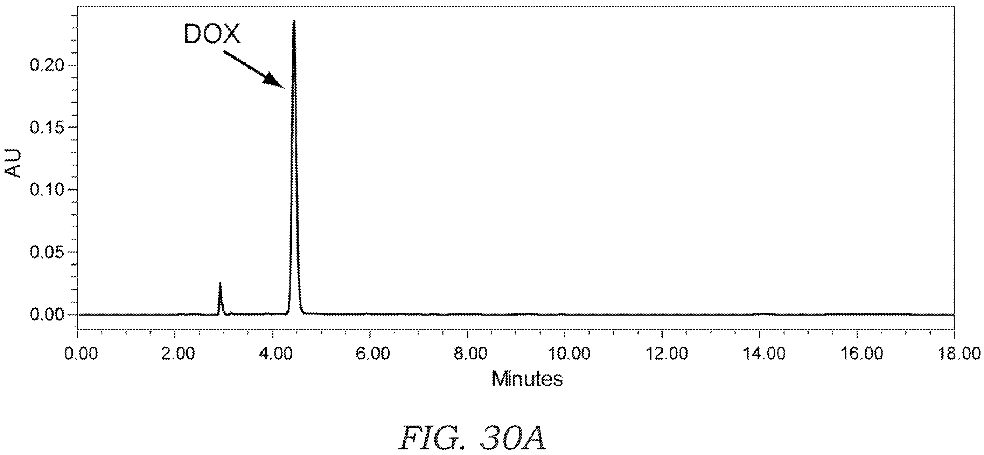

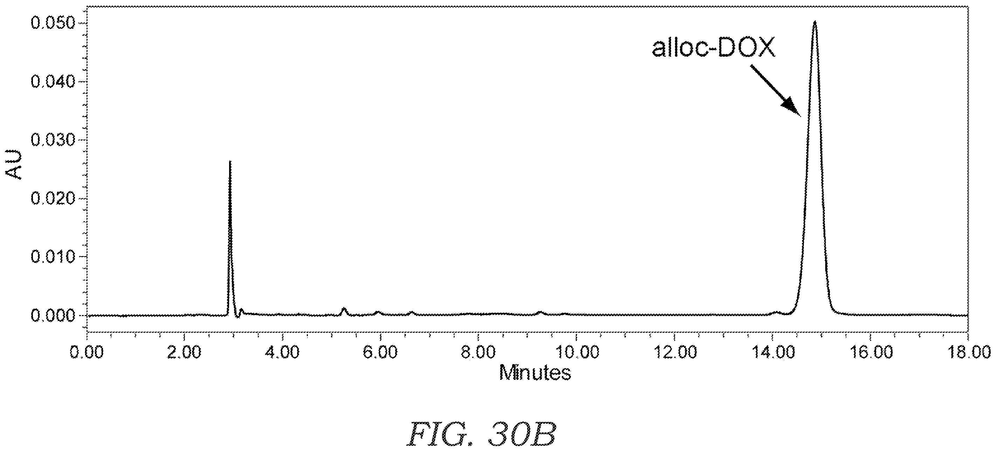

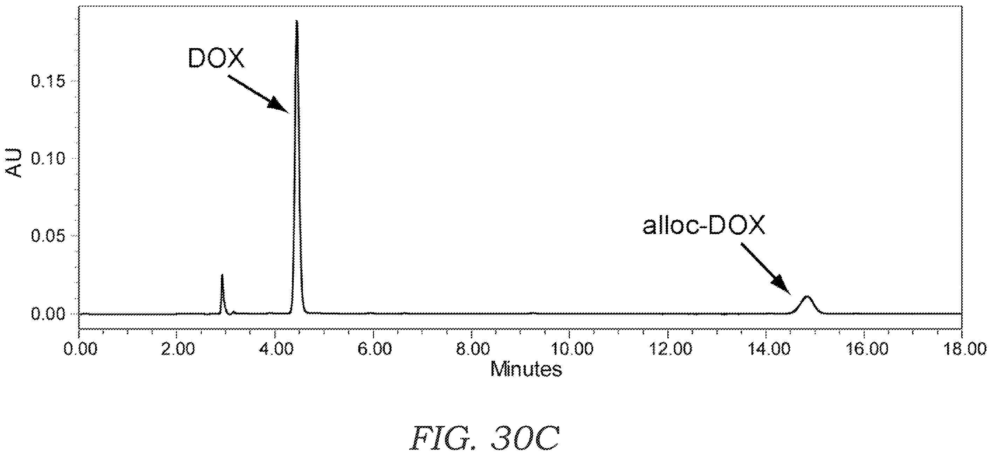

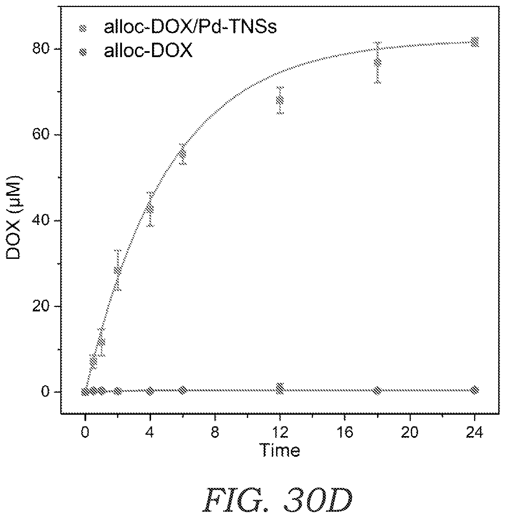

***In vitro* prodrug activation by PT-MNs.** Next, prodrug activation by PT-MNs 10 for cancer therapy was investigated. With the aim of confining cytotoxicity to the tumor site while diminishing damage toward normal tissues 100 during treatment, various bioliable prodrugs have been developed by leveraging the metabolic or physiological aberrations within cancerous tissues. Yet, their efficiency is often hampered by the heterogeneity nature of tumors. In this regard, bioorthogonal catalysis would provide an alternative promising strategy for spatial and temporal activation of prodrugs. For benchmarking, N-(allyloxycarbonyl)doxorubicin (alloc-DOX) was synthesized as a bioorthogonal prodrug (FIGS. 27, 28). Doxorubicin (DOX) is therapeutic agent/drug that is clinically used for cancer treatment which acts by binding DNA and eliciting enzyme-mediated strand breakage. Still, its full deployment is limited by side effects such as cardiotoxicity, myelosuppression, nausea, and vomiting. By caging the primary amine with allylcarbamate group, alloc-DOX (*K*_{alloc-DO*X*}= 7.96×10⁴ M⁻¹) showed a significantly lower binding affinity toward calf thymus DNA than DOX (*K*_{DO}*_{X}=* 9.36×10⁵ M⁻¹), as revealed by fluorescence titration method (FIGS. 5A, 5B and FIGS. 29A-29D). This weaker DNA binding ability of the alloc-DOX could be due to that the reduction in positive charge of the daunosamine moiety reduced the stability of DNA-drug intercalating complex. Meanwhile, the decaging of alloc-DOX catalyzed by Pd-TNSs 26 in solution was proved by high performance liquid chromatography (HPLC) analysis (FIGS. 30A-30D). Such masking strategy would afford a less toxic prodrug while allow activation by bioorthogonal catalysis.

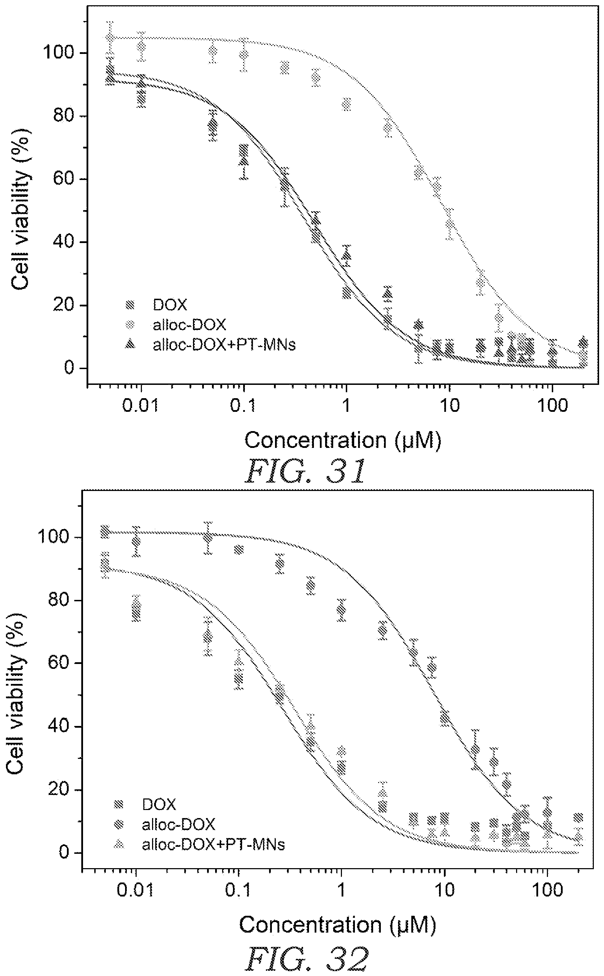

Next, a cell viability assay using B16F10 cells was carried out to study the toxicity profiles of alloc-DOX, DOX and alloc-DOX/PT-MNs 10 combination at different concentrations from 5 nM to 0.2 mM. Compared to parent DOX which showed elevated toxicity as its concentration increased, alloc-DOX displayed much lower antiproliferative activity (FIG. 5C). It was calculated that alloc-DOX (IC₅₀ = 9.29 µM) was nearly 120-times less toxic than DOX (IC₅₀ = 0.08 µM). In contrast, the introduction of PT-MNs 10 into the culture medium containing alloc-DOX led to a distinct decrease in cell viability at each concentration with an IC₅₀ of 0.12 µM, which was comparable to the trend manifested by DOX. In parallel, similar results were observed when the same experiments were performed with other cell lines, such as HepG2 (FIG. 31) and 4T1 cells (FIG. 32).

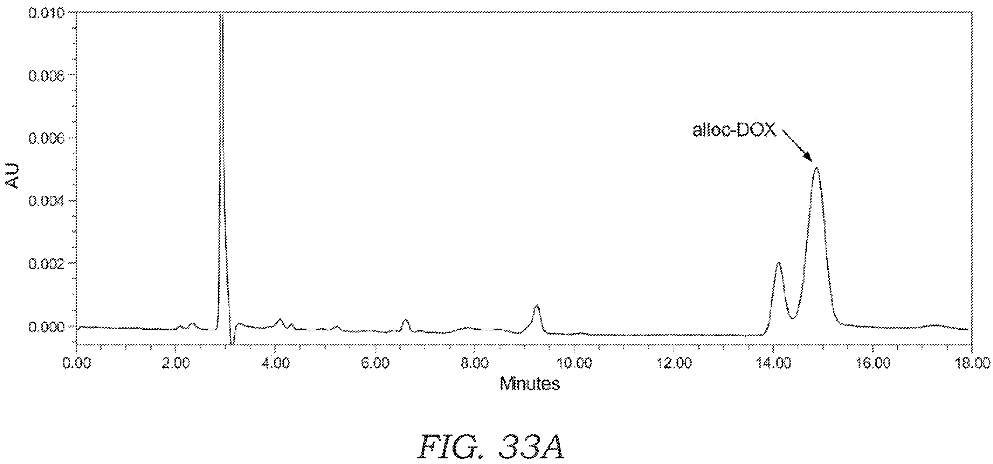

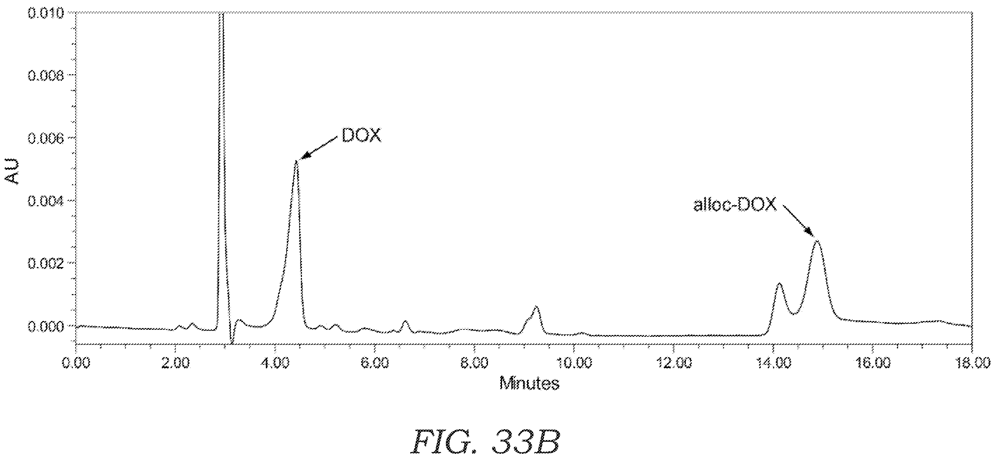

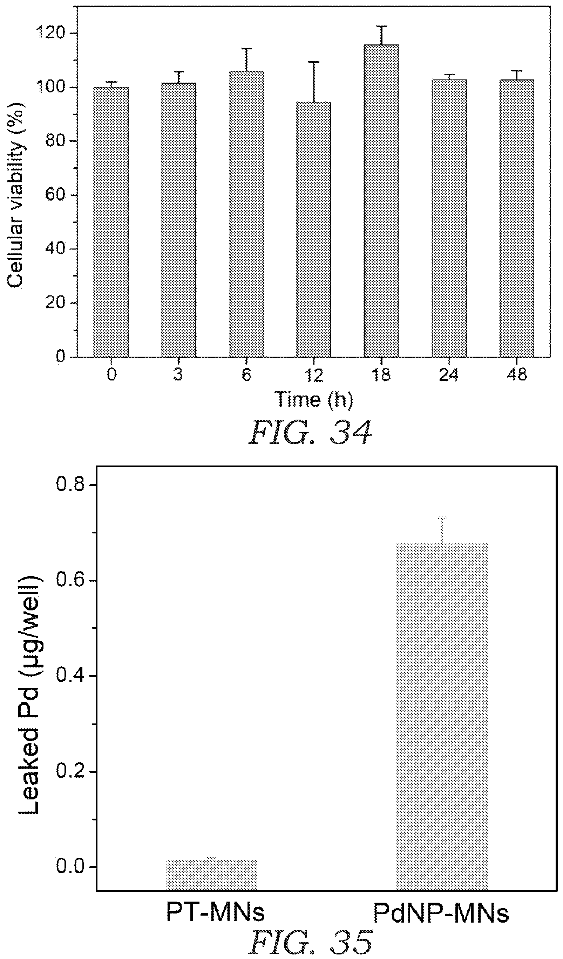

Analysis of the culture medium immersed with PT-MNs 10 by HPLC verified the extracellular generation of DOX (FIGS. 33A-33B). Furthermore, by dually staining the cells with Annexin V-APC (allophycocyanin) and SYTOX Green and analyzing with flow cytometry (FIG. 5D), a high proportion (44.1%) of the cell population treated with alloc-DOX/PT-MNs 10 combination were Annexin V-APC positive, similar to the group treated with DOX (64.2%). Meanwhile, no significant apoptotic cell death was observed in the control groups treated with alloc-DOX or PT-MNs 10 alone. Additionally, different from control cells which grow exuberantly, cells in the groups treated with alloc-DOX/PT-MNs combination or DOX showed extensive apoptotic DNA fragmentation, as revealed by terminal deoxynucleotidyl transferase dUTP nick end labelling (TUNEL) assay (FIG. 5E). All these results agree well with the features of apoptosis triggered by DOX, corroborating that the cytotoxicity induced by the alloc-DOX/PT-MNs 10 combination was due to the bioorthogonal generation of DOX in cell culture. Moreover, it is worth mentioning that the PT-MNs 10 at zero prodrug concentration showed no toxicity towards the cells after different time exposure (FIG. 34) and negligible leaked Pd was detected at the end (FIG. 35), which could be attributed to the stability of the patch 10 as discussed above.

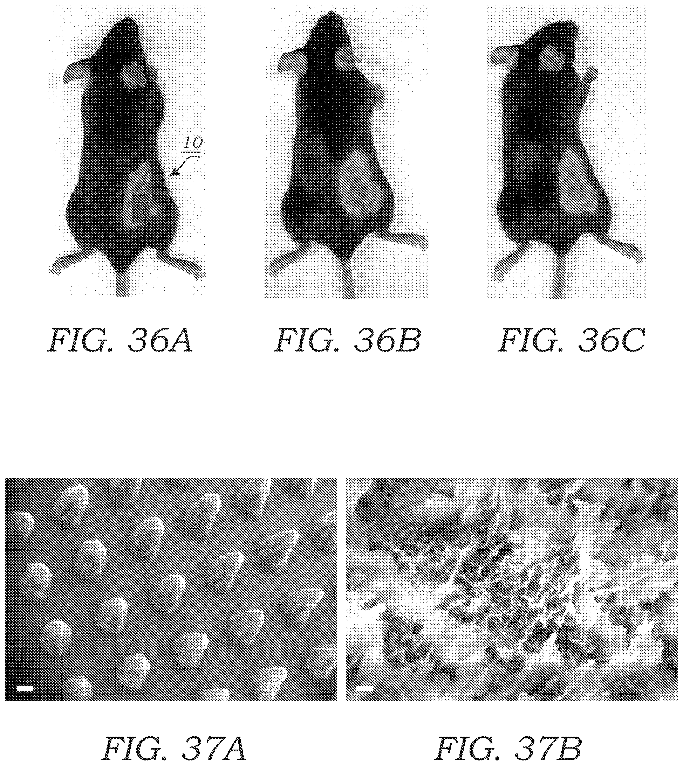

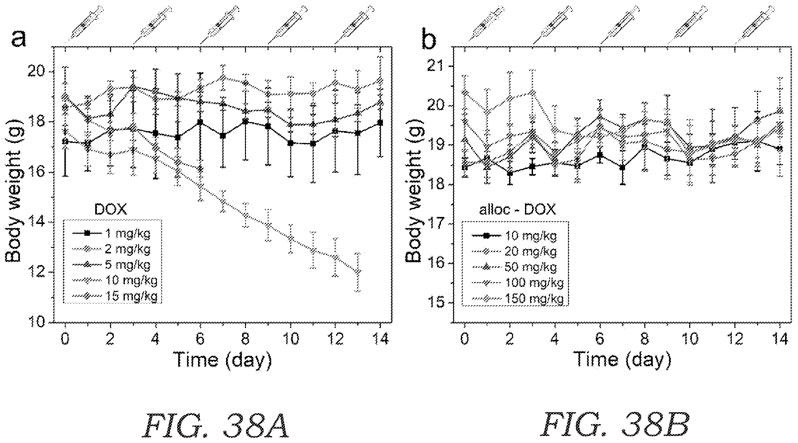

***In vivo* prodrug activation by PT-MNs for cancer therapy.** To evaluate the *in vivo* tumor inhibition performance by the bioorthogonal PT-MNs/alloc-DOX combination, an anticancer study was undertaken using a B16F10 mouse melanoma model. First, the ability of PT-MNs 10 to penetrate skin tissue 100 was evidenced by pressing the patch 10 with a gentle force against the skin 100 over the melanoma tumor (FIGS. 36A-36C). It could be seen that the needle 14 matrix swelled into three-dimensional porous networks after removing from mice (FIGS. 37A-37B), such changes in structure could help to alleviate mass transport barriers during the bioorthogonal catalysis. Meanwhile, to decide which the dose of alloc-DOX could be given for treatment, the *in vivo* toxicity of alloc-DOX was determined by measuring the weight changes of mice and compared with those of mice treated with DOX (FIGS. 38A-38B). Intraperitoneal (*i.p*.) injection of alloc-DOX up to 150 mg/kg every three (3) days led to weight loss of ~7.7% meanwhile serious weight loss and death were caused by DOX when its dose was over 10 mg/kg, suggesting the low toxicity of the prodrug *in vivo.* Based on these data, 100 mg/kg alloc-DOX and 5 mg/kg DOX were applied in the following antitumor studies.

Next, B16F10 tumor-bearing mice were randomly divided into five groups and treated with alloc-DOX/PT-MNs 10 combination, DOX, alloc-DOX, PT-MNs 10 (without prodrug) and PBS, respectively, once every 3 days. DOX, alloc-DOX and PBS were administered by *i.p.* injection. Before starting injection of prodrugs, PT-MNs 10 were inserted into the tumor site from the surface skin and fixed there for one (1) hour to allow the needles 14 to swell thoroughly. Bioluminescence imaging and tumor volumes were recorded to visualize tumor growth and evaluate the therapy efficiency (FIG. 6A). As shown in FIGS. 6A-6C, like the control group treated with PBS, no delayed tumor growth was observed for groups treated with alloc-DOX or PT-MNs 10 alone, indicating that neither the prodrug nor the catalytic alone had antitumor effect. Moderate inhibitory outcome was observed in the DOX-treated group, but the tumors grew quickly after DOX administration was stopped. By significant contrast, the alloc-DOX/PT-MNs 10 combination suppressed tumor growth much better, leading to the smallest tumors in mice after four (4) times treatment. In addition, no obvious body weight fluctuation was observed for the mice in all groups (FIG. 6D), indicating the low side effects of the prodrug, PT-MNs 10 device and their combination. Importantly, the TUNEL assay verified apoptotic signals only in the groups treated with alloc-DOX/PT-MNs 10 and DOX, implying similar tumor cell killing mechanism (FIG. 6E).

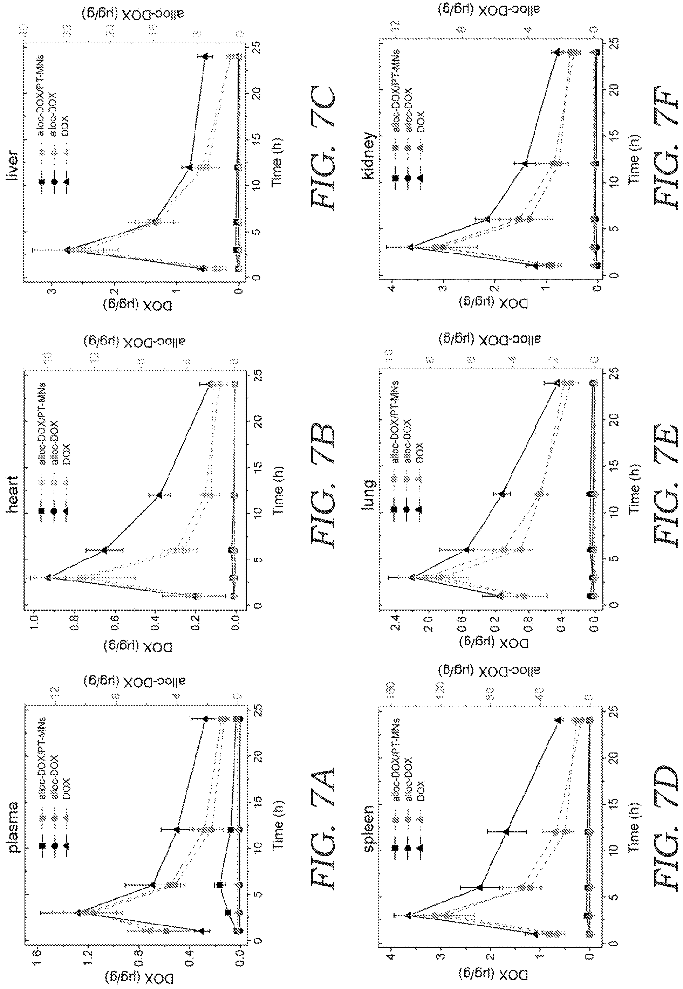

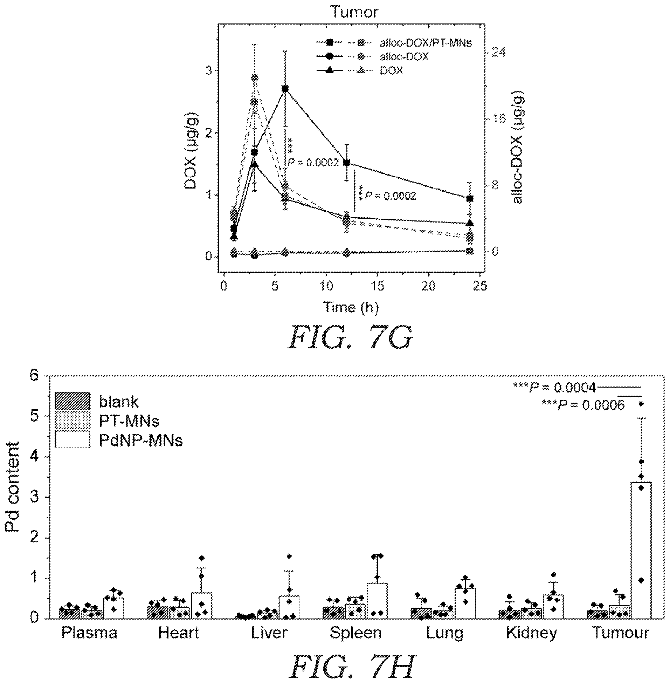

To gain further insights into these treatment results, the concentrations of drug/prodrug in various tissues, including tumor, plasma, and other major organs, versus time after different treatments were analyzed. As shown in FIGS. 7A-7G, no DOX was detected in all tissues in the group treated with alloc-DOX alone, indicating the stability of the prodrug against unexpected activation by high-level biological metabolic processes. In comparison with the group treated with DOX, there was more alloc-DOX than DOX in all tissues, which was due to that the dose of prodrug was allowed to be given higher. As for the alloc-DOX/PT-MNs 10 combination-treated group, the profiles of alloc-DOX concentration in plasma and other normal organs were similar to those in the group treated with alloc-DOX alone. However, the generation of DOX within the tumor tissues from alloc-DOX in the presence of PT-MNs 10 was validated, and, notably, the concentration of DOX was much higher at 6 and 12 h post i.p. injection than that of DOX-treated group. Also, importantly, there was no obvious leakback of the generated DOX from tumor site to the blood stream or other major organs. This might be attributed to the three-dimensional configuration of PT-MNs 10 that facilitated the uniform diffusion of the generated DOX within tumors and thereby promote their uptake by cancer cells. The small amount of DOX measured in some tissues might originate from the DOX generated by the part of microneedles 14 that stride between the tumor area and the skin. Collectively, it can be concluded that the accumulation of alloc-DOX inside tumors and its locoregional activation by the bioorthogonal catalytic patch 10 restored the drug's pharmacological property to combat tumors from within while minimizing side toxicity towards distant organs and tissues.

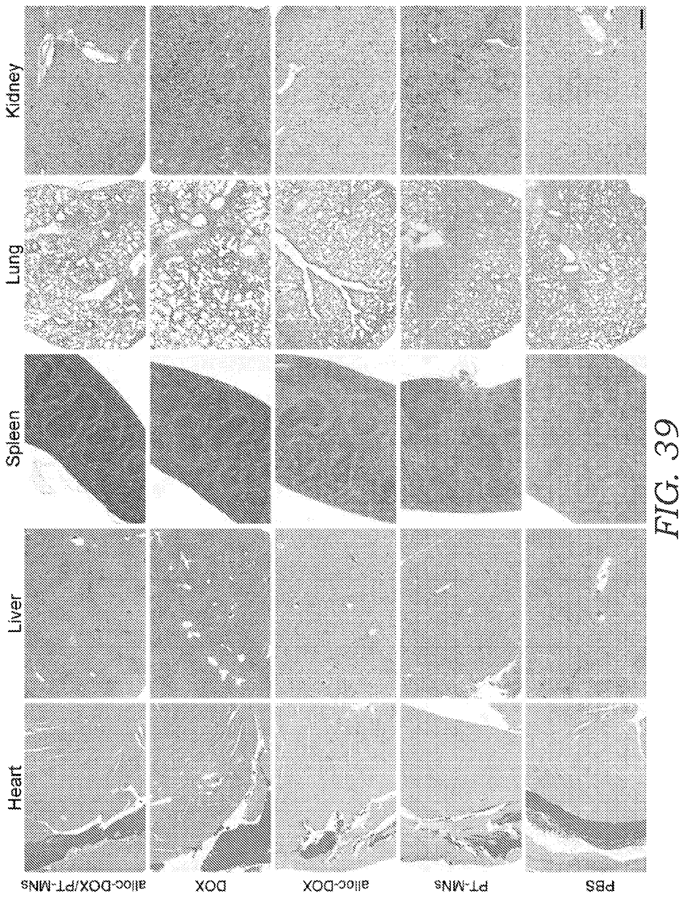

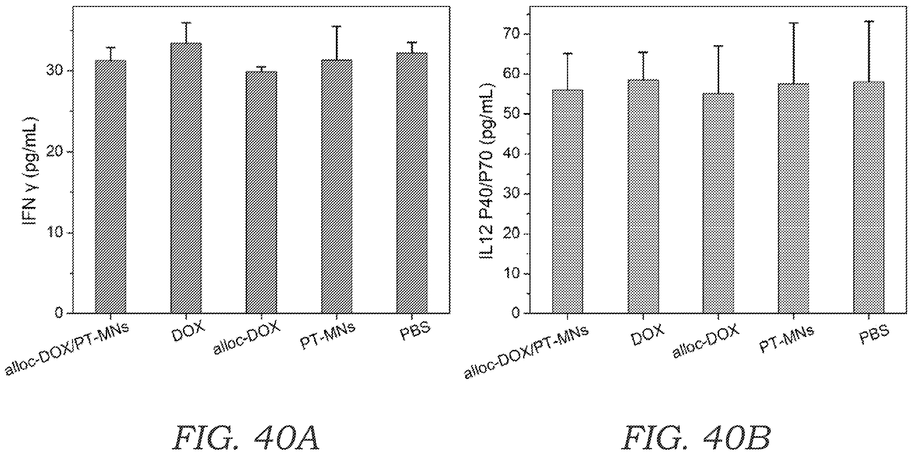

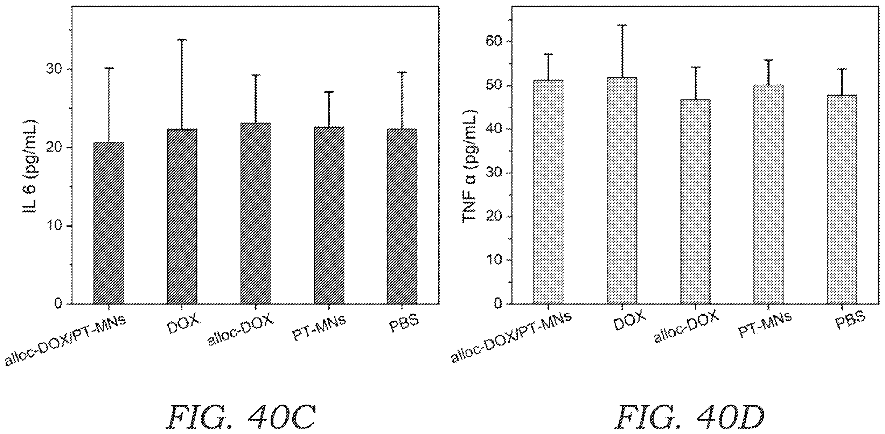

Lastly, but importantly, the PT-MNs 10 could be withdrawn without notable damage in the hydrated state after treatment (FIG. 37A), which could be attributed that the hydrogen bonds between PVA chains and the interfacial interaction between TNS 28 and PVA conferred the swollen PT-MNs 10 with enough mechanical strength. Analysis of the Pd content in the PT-MNs-inserted tumor tissue part, plasma, or other major organs showed no differences when compared with the blank control groups, suggesting that the stability of the patch 10 did not allow catalyst leakage in living systems (FIG. 7H). Such complete removal of bioorthogonal catalysts in the form microneedle devices would relieve the concern of transition metal toxicity. Moreover, the temporal microholes gradually resealed over 2 h after removing the microneedles 14 (FIGS. 36B, 36C), which would reduce the chance of infection there. Hematoxylin and eosin (H&E) staining of the major organs revealed that no noticeable damage or inflammation for any group (FIG. 39). And, the levels of factors, such as TNF-α, IFN-y and IL-6, did not differ from those in the blank control group injected with PBS, indicating no inflammation was induced (FIGS. 40A-40D).

A microneedle-based catalytic patch 10 device and its application are disclosed for mediating bioorthogonal transformation of biologically innocuous substances into active therapeutic agents or drugs *in vitro* and *in vivo.* The patch 10 was built upon a polyvinyl alcohol matrix integrated with palladium nanoparticle-deposited TiO₂ nanosheets 28 as the nanofillers 26, where the favorable interfacial interactions give the microneedles 14 high mechanical strength in a dry state for skin penetration and good stability in hydrated state against unexpected transition metal leakage. As such, this bioorthogonal patch 10 device showed high stability, good biocompatibility, easy removability and could effectively convert inert substrates into their parental states in aqueous solution, extracellular space and a selected area of tissue. The patch 10 enabled activation of a caged anticancer drug at tumor site and restoration of its pharmacological properties in a spatiotemporally controlled manner, by which off-targeted activation or dose-dependent side effects towards organs or tissues in distance were mitigated. This paves the way for locally interrogating the biological processes of a defined area of interest in high-level livings with bioorthogonal chemistry.

The development of readily implantable and removable microneedle-based patch 10 supports the transition towards safer bioorthogonal catalytic chemistry without metal deposition or causing inflammation. Either metal complexes or particulate metals still faces challenges of noncontrolled metal release, non-specific absorption during circulation, uncertain metal toxicity or clearance by the biological systems, which not only affects their own effectiveness but also can trigger off-targeted prodrug activation. Also, additional labor is needed to optimize the time lag between the sequential administration of catalysts and prodrugs, study the biological distribution of catalysts, and surgically transplant and/or withdraw the catalysts at the end. All these issues imply that achieving transition metalcatalyzed conversions of exogenous substrates within living systems is challenging. Conversely, by integrating microneedle array patches 10 with biorthogonal functionality, microneedle patches 10 are designed for extremely localized bioorthogonal chemistry to a specific area of tissue, further strengthening the orthogonality of abiotic chemistry. Moreover, such confined nature only requires minimally invasive transdermal application, the operation of which can be conducted with simple training. Along with the being expanded development of microneedles and bioorthogonal concept, it can be naturally envisioned that the device or patch 10 is modular and scalable, where the transition metal catalysts, the mediated chemical reactions (prodrugs), and the microneedle matrix and array patterns can be flexibly modulated and optimized for treating a variety of diseases, not only associated with skin, but also for other organs and tissue types.

While embodiments of the present invention have been shown and described, various modifications may be made without departing from the scope of the present invention. The invention, therefore, should not be limited, except to the following claims.

## Claims

1. A patch (10) for *in vivo* biorthogonal catalysis applications in mammalian tissue comprising:
a base or substrate (12) having a plurality of microneedles (14) extending away from the surface of the base (12), wherein the plurality of microneedles (14) comprise a polyvinyl alcohol (PVA) matrix having TiO₂ nanosheets (28) populated with metallic nanoparticles (30) dispersed therein.

2. The patch (10) of claim 1, wherein the metallic nanoparticles (30) comprise palladium or an alloy or metal complex thereof.

3. The patch (10) of claim 1, wherein the metallic nanoparticles (30) comprise gold, ruthenium, iridium, or copper or an alloy or metal complex thereof.

4. The patch (10) of claim 1, further comprising a backing material (20) on the base or substrate (12).

5. The patch (10) of claim 1, wherein the metallic nanoparticles (30) have an average size of less than 50 nm.

6. The patch (10) of claim 1, wherein the metallic nanoparticles (30) have an average size of less than 10 nm.

7. The patch (10) of claim 1, wherein the TiO₂ nanosheet-supported metallic nanoparticles (26) comprise between about 2 wt% and about 10 wt% of the patch.

8. The patch (10) of claim 1, wherein the TiO₂ nanosheet-supported metallic nanoparticles (30) are uniformly dispersed in the microneedles (14).

9. The patch (10) of claim 1, wherein the patch is in a substantially dry state.

10. The patch (10) of claim 1, further comprising one or more prodrugs contained in the plurality of microneedles (14).

11. A therapeutic system for treating mammalian tissue comprising:
a base or substrate (12) having a plurality of microneedles (14) extending away from the surface of the base (12), wherein the plurality of microneedles (14) comprise a polyvinyl alcohol (PVA) matrix having TiO₂ nanosheets (28) populated with metallic nanoparticles (30) dispersed therein; and
a prodrug, wherein the prodrug is converted to a therapeutic agent or drug by the TiO₂ nanosheets (28) populated with metallic nanoparticles (30).

12. The system of claim 11, wherein the prodrug comprises N-(allyloxycarbonyl)doxorubicin (alloc-DOX) and the therapeutic agent or drug comprises doxorubicin (DOX).

13. The therapeutic system of claim 11, wherein the prodrug is contained in the plurality of microneedles.

14. The therapeutic system of claim 11, wherein the prodrug comprises an injectable material.

15. The therapeutic system of claim 11, wherein the TiO2 nanosheet (28)-supported metallic nanoparticles (30) comprise between about 2 wt% and about 10 wt% of the patch.

## Patentansprüche

1. Ein Pflaster (10) für *in vivo* biorthogonale Katalyse Anwendungen im Säugetiergewebe, umfassend:
eine Basis oder ein Substrat (12) mit einer Vielzahl von Mikronadeln (14), die sich von der Oberfläche der Basis (12) weg erstrecken, wobei die Vielzahl von Mikronadeln (14) eine Polyvinylalkohol (PVA)-Matrix mit TiO₂-Nanosheets (28) umfasst, die mit darin dispergierten metallischen Nanopartikeln (30) besetzt sind.

2. Das Pflaster (10) nach Anspruch 1, wobei die metallischen Nanopartikel (30) Palladium oder eine Legierung oder einen Metallkomplex davon umfassen.

3. Das Pflaster (10) nach Anspruch 1, wobei die metallischen Nanopartikel (30) Gold, Ruthenium, Iridium oder Kupfer oder eine Legierung oder einen Metallkomplex davon umfassen.

4. Das Pflaster (10) nach Anspruch 1, ferner umfassend ein Trägermaterial (20) auf der Basis oder dem Substrat (12).

5. Das Pflaster (10) nach Anspruch 1, wobei die metallischen Nanopartikel (30) eine durchschnittliche Größe von weniger als 50 nm haben.

6. Das Pflaster (10) nach Anspruch 1, wobei die metallischen Nanopartikel (30) eine durchschnittliche Größe von weniger als 10 nm haben.

7. Das Pflaster (10) nach Anspruch 1, wobei die TiO₂-Nanosheet-unterstützten metallischen Nanopartikel (26) zwischen etwa 2 Gewichtsprozent und etwa 10 Gewichtsprozent des Pflasters umfassen.

8. Das Pflaster (10) nach Anspruch 1, wobei die TiO₂-Nanosheet-unterstützten metallischen Nanopartikel (30) gleichmäßig in den Mikronadeln (14) dispergiert sind.

9. Das Pflaster (10) nach Anspruch 1, wobei das Pflaster in einem im Wesentlichen trockenen Zustand ist.

10. Das Pflaster (10) nach Anspruch 1, ferner umfassend ein oder mehrere Prodrugs, die in der Vielzahl von Mikronadeln (14) enthalten sind.

11. Ein therapeutisches System zur Behandlung von Säugetiergewebe, umfassend:
eine Basis oder ein Substrat (12) mit einer Vielzahl von Mikronadeln (14), die sich von der Oberfläche der Basis (12) weg erstrecken, wobei die Vielzahl von Mikronadeln (14) eine Polyvinylalkohol (PVA)-Matrix mit TiO₂-Nanosheets (28) umfasst, die mit darin dispergierten metallischen Nanopartikeln (30) besetzt sind; und
ein Prodrug, wobei das Prodrug durch die TiO₂-Nanosheets (28), die mit metallischen Nanopartikeln (30) besetzt sind, in ein therapeutisches Mittel oder Medikament umgewandelt wird.

12. Das System nach Anspruch 11, wobei das Prodrug N-(allyloxycarbonyl)doxorubicin (alloc-DOX) umfasst und das therapeutische Mittel oder Medikament Doxorubicin (DOX) umfasst.

13. Das therapeutische System nach Anspruch 11, wobei das Prodrug in der Vielzahl von Mikronadeln enthalten ist.

14. Das therapeutische System nach Anspruch 11, wobei das Prodrug ein injizierbares Material umfasst.

15. Das therapeutische System nach Anspruch 11, wobei die TiO₂-Nanosheet (28)-unterstützten metallischen Nanopartikel (30) zwischen etwa 2 Gewichtsprozent und etwa 10 Gewichtsprozent des Pflasters umfassen.

## Revendications

1. Un patch (10) pour des applications de catalyse biorthogonale *in vivo* dans les tissus mammifères comprenant :
une base ou un substrat (12) ayant une pluralité de microaiguilles (14) s'étendant à partir de la surface de la base (12), où la pluralité de microaiguilles (14) comprend une matrice de polyalcool vinylique (PVA) ayant des nanofeuilles de TiO₂ (28) peuplés de nanoparticules métalliques (30) dispersées à l'intérieur.

2. Le patch (10) de la revendication 1, où les nanoparticules métalliques (30) comprennent du palladium ou un alliage ou complexe métallique de celui-ci.

3. Le patch (10) de la revendication 1, où les nanoparticules métalliques (30) comprennent de l'or, du ruthénium, de l'iridium ou du cuivre ou un alliage ou complexe métallique de ceux-ci.

4. Le patch (10) de la revendication 1, comprenant en outre un matériau de support (20) sur la base ou le substrat (12).

5. Le patch (10) de la revendication 1, où les nanoparticules métalliques (30) ont une taille moyenne inférieure à 50 nm.

6. Le patch (10) de la revendication 1, où les nanoparticules métalliques (30) ont une taille moyenne inférieure à 10 nm.

7. Le patch (10) de la revendication 1, où les nanoparticules métalliques supportées par des nanofeuilles de TiO₂ (26) comprennent entre environ 2 % en poids et environ 10 % en poids du patch.

8. Le patch (10) de la revendication 1, où les nanoparticules métalliques supportées par des nanofeuilles de TiO₂ (30) sont uniformément dispersées dans les microaiguilles (14).

9. Le patch (10) de la revendication 1, où le patch est dans un état substantiellement sec.

10. Le patch (10) de la revendication 1, comprenant en outre une ou plusieurs prodrogues contenues dans la pluralité de microaiguilles (14).

11. Un système thérapeutique pour traiter les tissus mammifères comprenant :
une base ou un substrat (12) ayant une pluralité de microaiguilles (14) s'étendant à partir de la surface de la base (12), où la pluralité de microaiguilles (14) comprend une matrice de polyalcool vinylique (PVA) ayant des nanofeuilles de TiO₂ (28) peuplés de nanoparticules métalliques (30) dispersées à l'intérieur ; et
une prodrogue, où le prodrogue est converti en un agent thérapeutique ou médicament par les nanofeuilles de TiO₂ (28) peuplés de nanoparticules métalliques (30).

12. Le système de la revendication 11, où la prodrogue comprend de la N-(allyloxycarbonyl)doxorubicine (alloc-DOX) et l'agent thérapeutique ou médicament comprend de la doxorubicine (DOX).

13. Le système thérapeutique de la revendication 11, où la prodrogue est contenu dans la pluralité de microaiguilles.

14. Le système thérapeutique de la revendication 11, où la prodrogue comprend un matériau injectable.

15. Le système thérapeutique de la revendication 11, où les nanoparticules métalliques supportées par des nanofeuilles de TiO₂ (28) comprennent entre environ 2 % en poids et environ 10 % en poids du patch.
